# EUROPEAN PATENT APPLICATION

(11) **EP 4 162 953 A1**
(43) Date of publication of application: **12.04.2023**
(21) Application number: 21817415.9
(22) Date of filing: 01.06.2021
(51) Int. Cl.: A61K 45/00, A61K 31/27, A61P 1/00, A61P 37/02

(54) **THERAPEUTIC DRUG FOR DISEASE CAUSED BY INTESTINAL IMMUNE DISORDER**

(30) Priority: 01.06.2020 JP 2020095241
(71) Applicant: Keio University, Tokyo, 108-8345 (JP)
(72) Inventor: KANAI, Takanori, Tokyo 160-8582 (JP); TERATANI, Toshiaki, Tokyo 160-8582 (JP); MIKAMI, Yohei, Tokyo 160-8582 (JP)
(74) Representative: J A Kemp LLP
(86) International application number: PCT/JP2021/020821
(87) International publication number: WO 2021/246399

(57) **Abstract**

An object of the present invention is to regulate the number of peripheral regulatory T cells in the gut and establish a novel therapeutic approach for a disease related to the cells. The present invention provides a therapeutic agent for a disease, comprising a substance having an effect of regulating an amount of peripheral regulatory T cells in the gut, wherein the substance is a substance activating or inhibiting afferent pathway of the hepatic branch of the vagus nerve, a substance activating or inhibiting efferent pathway of the left vagus nerve, or an agonist or an antagonist of a muscarinic acetylcholine receptor.

## Description

### [Technical Field]

The present invention relates to a novel therapeutic agent for a disease, a novel method for screening for a therapeutic agent for a disease, and a novel method for treating a disease.

### [Background Art]

The gut functions as an important organ responsible for digestion and absorption and faces the outside world (lumen) through a single layer of columnar epithelial cells. The gut homeostasis is maintained so as not to cause excessive inflammatory response by the functions of peripheral regulatory T cells (pTregs) in the gut, though continuously exposed to more than one hundred trillion foreign matters such as intestinal bacteria and food antigens in the lumen. For the differentiation and maintenance of peripheral regulatory T cells, particular intestinal bacteria, intestinal bacteria derived components, short chain fatty acids, cytokines, and the like have been emphasized so far. Meanwhile, relatively high incidences of inflammatory bowel diseases in depression or irritable bowel syndrome considered as nerve disease have suggested the possibility that the autonomic nerve is deeply involved in the abnormal immunity of the gut (Non Patent Literatures 1, 2, 3, and 4). Although recent reports have suggested possible involvement of the nervous system in the gut immune mechanism, the relationship between the nervous system and peripheral regulatory T cells in the gut has been unknown for a long period. Previous study reports on brain-gut interaction do not specifically show any nerve circuit that connects the brain and the gut, and a lot of questions have also remained from the anatomical standpoint.

### [Citation List]

### [Non Patent Literature]

[Non Patent Literature 1] Godinho-Silva, C., Cardoso, F. & Veiga-Fernandes, H. Neuro-Immune Cell Units: A New Paradigm in Physiology. Annu. Rev. Immunol. 37, 19-46 (2019).
[Non Patent Literature 2] Chavan, S. S., Pavlov, V. A. & Tracey, K. J. Mechanisms and Therapeutic Relevance of Neuro-immune Communication. Immunity 46, 927-942 (2017).
[Non Patent Literature 3] Huh, J. R. & Veiga-Fernandes, H. Neuroimmune circuits in inter-organ communication. Nat Rev Immunol 20, 217-228 (2019).
[Non Patent Literature 4] Chu, C., Artis, D. & Chiu, I. M. Neuro-immune Interactions in the Tissues. Immunity 52, 464-474 (2020).

### [Summary of Invention]

### [Technical Problem]

If the relationship between the nervous system and peripheral regulatory T cells in the gut is revealed, the number of peripheral regulatory T cells in the gut can be artificially regulated, also leading to the development of novel methods for treating diseases related to peripheral regulatory T cells in the gut (e.g., inflammatory bowel diseases).

The present invention has been made against this backdrop, and an object of the present invention is to regulate the number of peripheral regulatory T cells in the gut and provide a novel therapeutic approach for a disease related to the cells.

### [Solution to Problem]

The present inventor has conducted diligent studies to attain the object and consequently found that: 1) peripheral regulatory T cells in the gut are influenced by parasympathetic signals from the brain; 2) the liver accumulates and combines information of the gut environment and transmits the information of the gut environment to the brain via the pathways of the vagus nerve towards the brain; and 3) antigen-presenting cells, which are reportedly very important for the differentiation and maintenance of peripheral regulatory T cells in the gut, are positioned approximally to the nerve in intestinal lamina propria mucosae and the intestinal antigen-presenting cells strongly express muscarinic acetylcholine receptor subtype 1.

The present invention has been completed on the basis of these findings.

Specifically, the present invention provides the following [1] to [20].
[1] A therapeutic agent for a disease, comprising a substance having an effect of regulating an amount of peripheral regulatory T cells in the gut, wherein the substance is a substance activating or inhibiting afferent pathway of the hepatic branch of the vagus nerve, a substance activating or inhibiting efferent pathway of the left vagus nerve, or an agonist or an antagonist of a muscarinic acetylcholine receptor.
[2] The therapeutic agent for a disease according to [1], wherein the disease is an inflammatory bowel disease, an autoimmune disease, an allergy, a cancer, depression, or a gastrointestinal infection.
[3] The therapeutic agent for a disease according to [1], wherein the disease is an inflammatory bowel disease.
[4] The therapeutic agent for a disease according to any of [1] to [3], wherein the substance having an effect of regulating an amount of peripheral regulatory T cells in the gut is an agonist of a muscarinic acetylcholine receptor.
[5] The therapeutic agent for a disease according to [4], wherein the agonist of a muscarinic acetylcholine receptor is bethanechol, muscarine, pilocarpine, or cevimeline.
[6] The therapeutic agent for a disease according to any of [1] to [3], wherein the substance having an effect of regulating an amount of peripheral regulatory T cells in the gut is an antagonist of a muscarinic acetylcholine receptor.
[7] The therapeutic agent for a disease according to [6], wherein the antagonist of a muscarinic acetylcholine receptor is atropine, tropicamide, oxybutynin, propiverine, tolterodine, solifenacin, or imidafenacin.
[8] A method for screening for a therapeutic agent for a disease, comprising the steps of: co-culturing intestinal antigen-presenting cells and CD4 positive T cells in the presence of a test substance; and detecting induction of regulatory T cells.
[9] The screening method according to [8], wherein the induction of regulatory T cells is detected by detection of expression of FoxP3.
[10] The screening method according to [8] or [9], wherein the disease is an inflammatory bowel disease, an autoimmune disease, an allergy, a cancer, depression, or a gastrointestinal infection.
[11] The screening method according to [8] or [9], wherein the disease is an inflammatory bowel disease.
[12] A method for treating a disease by regulating an amount of peripheral regulatory T cells in the gut, comprising activating or inhibiting afferent pathway of the hepatic branch of the vagus nerve in a subject to be treated, activating or inhibiting efferent pathway of the left vagus nerve in a subject to be treated, or administering an agonist or an antagonist of a muscarinic acetylcholine receptor to a subject to be treated.
[13] The method for treating a disease according to [12], wherein the disease is an inflammatory bowel disease, an autoimmune disease, an allergy, a cancer, depression, or a gastrointestinal infection.
[14] The method for treating a disease according to [12], wherein the disease is an inflammatory bowel disease.
[15] The method for treating a disease according to any of [12] to [14], comprising administering an agonist of a muscarinic acetylcholine receptor to a subject to be treated.
[16] The method for treating a disease according to [15], wherein the agonist of a muscarinic acetylcholine receptor is bethanechol, muscarine, pilocarpine, or cevimeline.
[17] The method for treating a disease according to any of [12] to [14], comprising administering an antagonist of a muscarinic acetylcholine receptor to a subject to be treated.
[18] The method for treating a disease according to [17], wherein the antagonist of a muscarinic acetylcholine receptor is atropine, tropicamide, oxybutynin, propiverine, tolterodine, solifenacin, or imidafenacin.
[19] The method for treating a disease according to any of [12] to [18], wherein the subject to be treated is a non-human animal.
[20] A method for operating a cuff electrode, comprising stimulating the hepatic branch of the vagus nerve to regulate an amount of peripheral regulatory T cells in the gut.

The present specification encompasses the contents described in the specification and/or drawings of the Japanese patent application (Japanese Patent Application No. 2020-095241), on which the priority of the present application is based.

### [Advantageous Effects of Invention]

The present invention provides a novel therapeutic agent for a disease, a novel method for screening for a therapeutic agent for a disease, and a novel method for treating a disease.

### [Brief Description of Drawings]

[Figure 1] Potential interaction between APCs and neurons in the gut.
[Figure 2] The hepatic vagal sensory afferent pathway is essential for NTS activation during colitis.
[Figure 3] The liver-brain-gut axis regulates colonic Treg homeostasis through muscarinic signaling in APC.
[Figure 4] Perturbation of hepatic vagal afferents exacerbates murine colitis in a muscarinic signaling dependent manner.
[Figure 5] Muscarinic signaling in colonic APC activates induction of Treg.
[Figure 6] Colitis activates liver-brain axis.
[Figure 7] Anatomy of the hepatic vagus nerve in mice.
[Figure 8] Effects of vagotomy on maintenance and stability of colonic pTreg.
[Figure 9] Afferent vagal, but not spinal cord, activation from the liver is involved in colonic Treg homeostasis.
[Figure 10] Hemi-subdiaphragmatic vagotomy revealed functional asymmetries of the vagus nerve.
[Figure 11] Effects of VGx and HVx on intrinsic enteric neuron.
[Figure 12] Effects of mAChR and α7nAChR on maintenance of colonic Treg.
[Figure 13] Effects of gut-microbiota on colonic Treg maintenance of the liver-brain-gut axis.
[Figure 14] Effects of HVx on colitis.
[Figure 15] Schema of vagal hepatic nerve stimulation (VHNS).
[Figure 16] Vagal hepatic nerve stimulation (VHNS) inhibits pathological condition of colitis in mice.
[Figure 17] Two-bottle preference assays using vagotomized mice.
[Figure 18] Regulation of pulmonary immune cells by hepatic branch of vagus nerve.
[Figure 19] Regulation of intestinal peristalsis by vagus nerve.

### [Description of Embodiments]

Hereinafter, the present invention will be described in detail.

### (1) Therapeutic agent

The therapeutic agent for a disease of the present invention is a therapeutic agent for a disease, comprising a substance having an effect of regulating an amount of peripheral regulatory T cells in the gut, wherein the substance is a substance activating or inhibiting afferent pathway of the hepatic branch of the vagus nerve, a substance activating or inhibiting efferent pathway of the left vagus nerve, or an agonist or an antagonist of a muscarinic acetylcholine receptor.

In the present specification, the phrase "regulating an amount of peripheral regulatory T cells in the gut" means increasing or decreasing an amount of peripheral regulatory T cells in the gut. By increasing the amount of peripheral regulatory T cells in the gut, excessive immune response is suppressed, and a therapeutic effect on inflammatory diseases or the like can be expected. On the other hand, by decreasing the amount of peripheral regulatory T cells in the gut, immune response is enhanced, and a therapeutic effect on gastrointestinal infections or the like can be expected.

When used for the purpose of increasing the amount of peripheral regulatory T cells in the gut, the therapeutic agent for a disease of the present invention contains a substance activating afferent pathway of the hepatic branch of the vagus nerve, a substance activating efferent pathway of the left vagus nerve, or an agonist of a muscarinic acetylcholine receptor. By contrast, when used for the purpose of decreasing the amount of peripheral regulatory T cells in the gut, the therapeutic agent for a disease of the present invention contains a substance inhibiting afferent pathway of the hepatic branch of the vagus nerve, a substance inhibiting efferent pathway of the left vagus nerve, or an antagonist of a muscarinic acetylcholine receptor.

The type of the disease is not particularly limited as long as the disease can be treated by the regulation (increase or decrease) of the amount of peripheral regulatory T cells in the gut. Specifically, examples of the disease that can be treated by increasing the amount of peripheral regulatory T cells in the gut can include diseases caused by abnormal gut immunity (inflammatory bowel diseases, autoimmune diseases, allergies, etc.), cancers, and depression. Examples of the disease that can be treated by decreasing the amount of peripheral regulatory T cells in the gut can include gastrointestinal infections and cancers. Examples of the gastrointestinal infections can include norovirus infection, rotavirus infection, and pathogenic E. coli colitis.

The substance activating or inhibiting afferent pathway of the hepatic branch of the vagus nerve, the substance activating or inhibiting efferent pathway of the left vagus nerve, or the agonist or the antagonist of a muscarinic acetylcholine receptor is not particularly limited as long as the substance has an effect of regulating an amount of peripheral regulatory T cells in the gut. Specific examples of the agonist of a muscarinic acetylcholine receptor can include, but are not limited to, bethanechol, muscarine, pilocarpine, and cevimeline. Specific examples of the antagonist of a muscarinic acetylcholine receptor can include, but are not limited to, atropine, tropicamide, oxybutynin, propiverine, tolterodine, solifenacin, and imidafenacin.

The therapeutic agent for a disease of the present invention can be prepared by formulating the substance having an effect of regulating an amount of peripheral regulatory T cells in the gut in accordance with a pharmaceutical method known in the art. Specifically, the therapeutic agent can be prepared as an injection (intraperitoneal, subcutaneous, intravenous, or intramuscular injection), intravenous fluids, a capsule, a solution, a suspension, an emulsion, or the like. For formulation, other components such as pharmacologically acceptable carriers may be contained therein. Examples of other components can include sterile water, saline, solvents, bases, emulsifiers, plant oils, suspending agents, surfactants, stabilizers, antiseptics, binders, diluents, tonicity agents, soothing agents, disintegrants, lubricants, buffers, coating agents, colorants, and other additives. These components can be used in appropriate combination.

The subject to be treated with the therapeutic agent for a disease of the present invention is typically a human and may be a non-human animal. Examples of the non-human animal can include mice, rats, hamsters, rabbits, cats, dogs, bovines, horses, pigs, sheep, and monkeys.

The dose of the therapeutic agent for a disease of the present invention can be appropriately determined depending on the type of the substance having an effect of regulating an amount of peripheral regulatory T cells in the gut, the type of the disease, a dosage form, an administration method, the age and body weight of the subject to be treated, etc. Specifically, for example, in the case of administering an agonist of a muscarinic acetylcholine receptor to a human, the daily dose is preferably 0.1 to 100 g, more preferably 0.1 to 10 g, per adult.

Examples of the method for administering the therapeutic agent for a disease of the present invention can include, but are not particularly limited to, intraperitoneal injection, subcutaneous injection, injection into the lymph, intravenous injection, and drip intravenous injection.

### (2) Screening method

The screening method of the present invention is a method for screening for a therapeutic agent for a disease, comprising the steps of: co-culturing intestinal antigen-presenting cells and CD4 positive T cells in the presence of a test substance; and detecting induction of regulatory T cells. The method for detecting the induction of regulatory T cells is not particularly limited and is preferably performed by a method of detecting expression of FoxP3. The disease can be the same as that mentioned above about the therapeutic agent.

### (3) Treatment method

The method for treating a disease of the present invention is a method for treating a disease by regulating an amount of peripheral regulatory T cells in the gut, comprising activating or inhibiting afferent pathway of the hepatic branch of the vagus nerve in a subject to be treated, activating or inhibiting efferent pathway of the left vagus nerve in a subject to be treated, or administering an agonist or an antagonist of a muscarinic acetylcholine receptor to a subject to be treated.

The activation or inhibition of afferent pathway of the hepatic branch of the vagus nerve can be performed by administering a substance having such an effect to a subject to be treated. In addition, the activation or inhibition can also be performed by electrically stimulating the afferent pathway of the hepatic branch of the vagus nerve, or cleaving the afferent pathway of the hepatic branch of the vagus nerve. Likewise, the activation or inhibition of efferent pathway of the left vagus nerve can be performed by administering a substance having such an effect to a subject to be treated, electrically stimulating the efferent pathway of the left vagus nerve, or cleaving the efferent pathway of the left vagus nerve.

The agonist or the antagonist of a muscarinic acetylcholine receptor, the disease, and the subject to be treated, etc. can be the same as those mentioned above about the therapeutic agent.

### (4) Method for operating cuff electrode

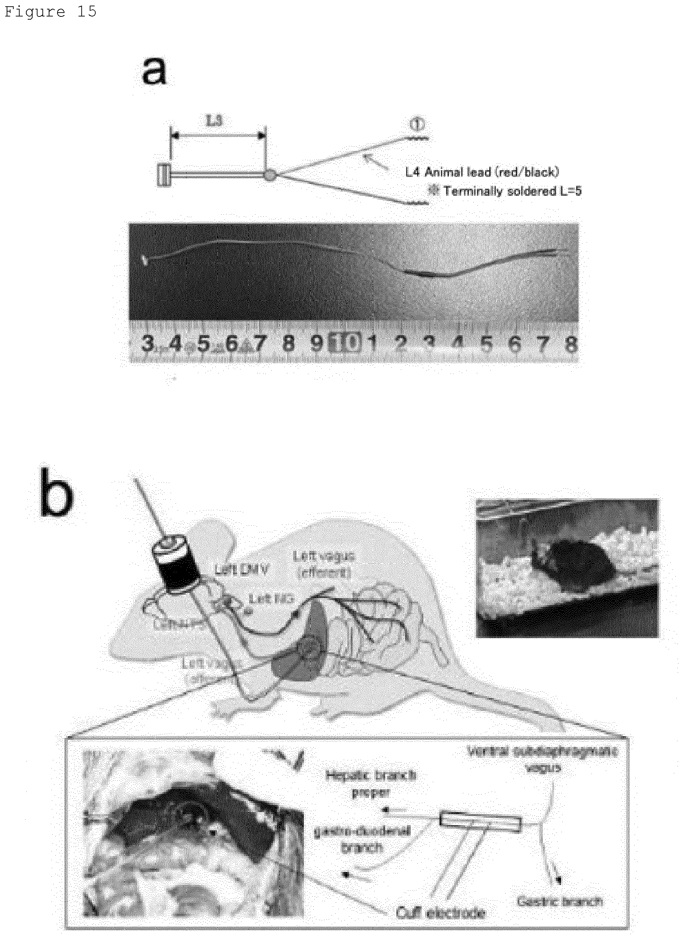

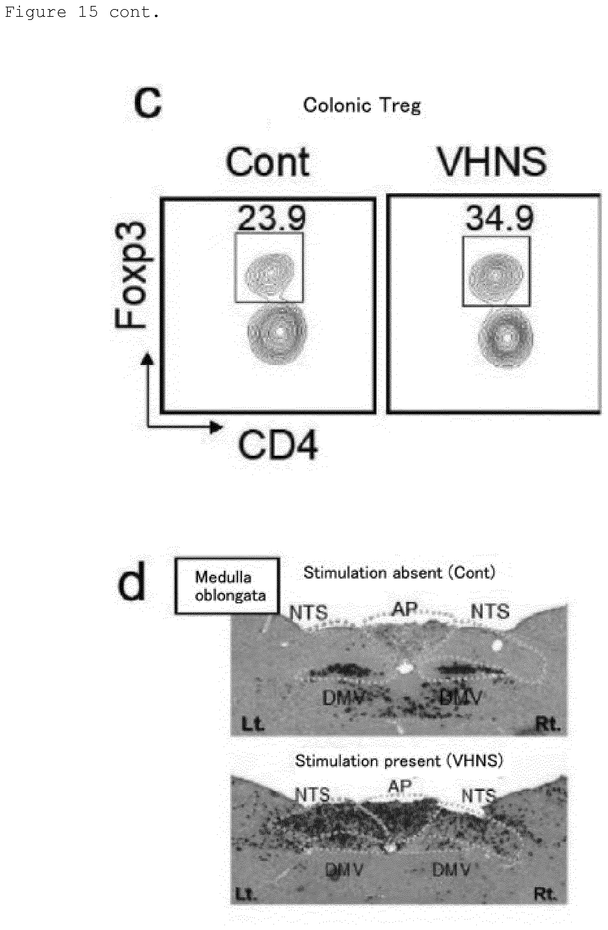

The method for operating a cuff electrode of the present invention comprises stimulating the hepatic branch of the vagus nerve to regulate an amount of peripheral regulatory T cells in the gut. As shown in Figure 15, the cuff electrode placed in the hepatic branch of the vagus nerve is operated so that the hepatic branch of the vagus nerve can be electrically stimulated to increase the amount of peripheral regulatory T cells in the gut. Diseases such as inflammatory bowel diseases can thereby be treated.

### [Examples]

Hereinafter, the present invention will be described in more detail with reference to Examples. However, the present invention is not limited by these Examples.

### [Example 1]

Foxp3⁺ peripheral regulatory T cells (pTregs) are most abundant in the mucosal tissues, especially the colonic lamina propria (LP), and maintain immune homeostasis in the gut ^{5,6}. The generation of pTregs is promoted by a combinations of cytokines, such as TGF-β and RA; microbial and dietary signals, such as *Clostridia* clusters IV, XIVa, and XVIII, *Bacteroides fragilis,* microbiota-associated molecular patterns (MAMPs), and short chain fatty acids (SCFA) ⁵⁻¹³. In addition to these numerous environmental stimuli, considerable advances have been made by recent studies showing that immune cells are under the control of autonomic and enteric neurons ^{3,14-16}. This suggests that pTreg differentiation at the mucosal sites is governed by previously unappreciated mechanisms.

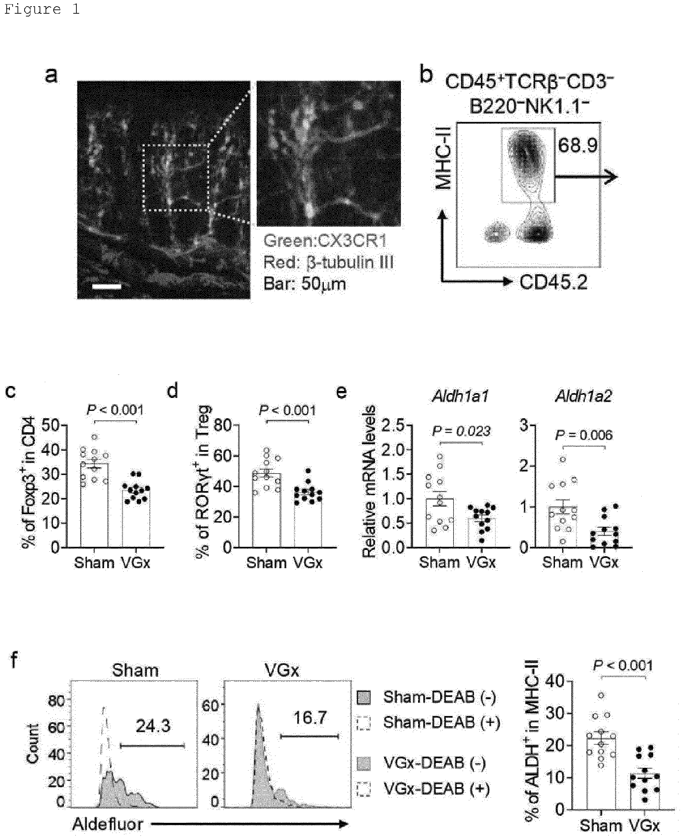

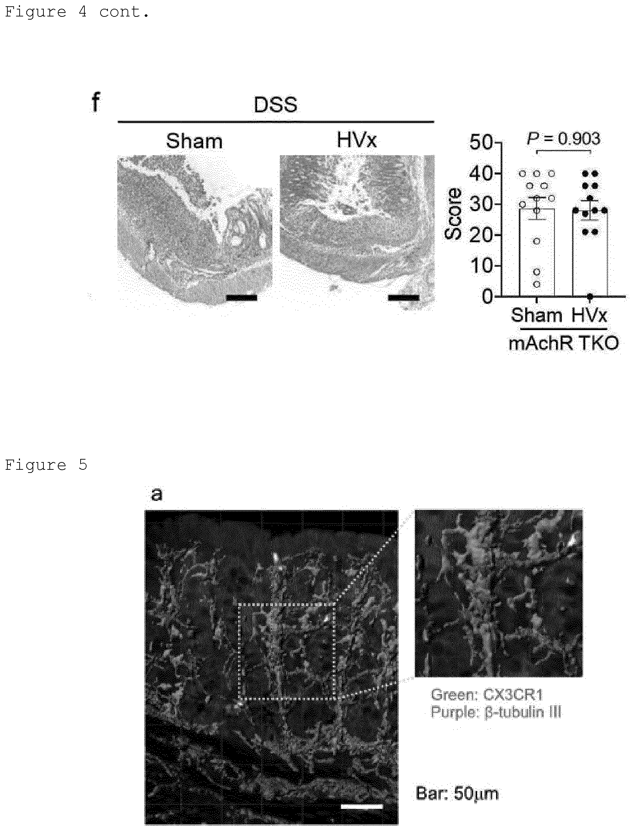

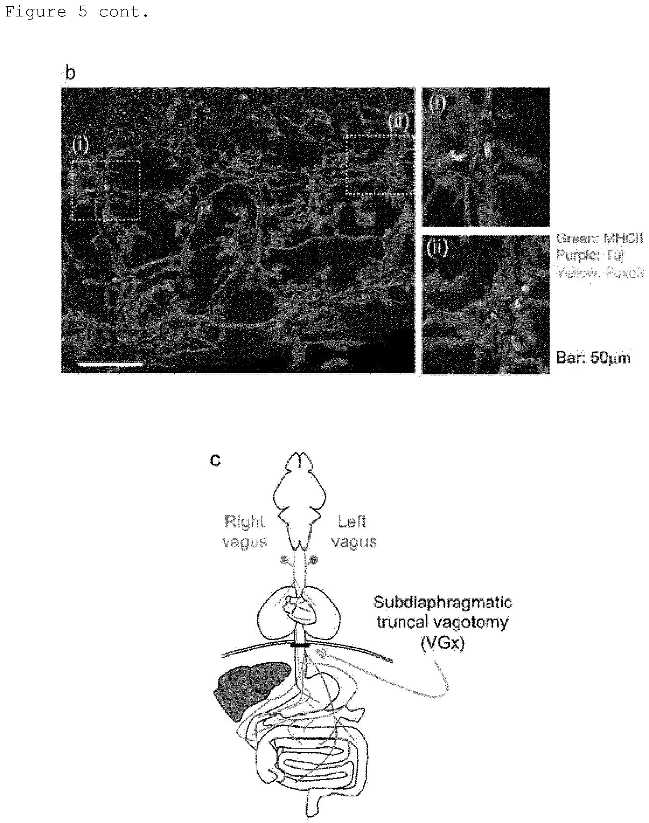

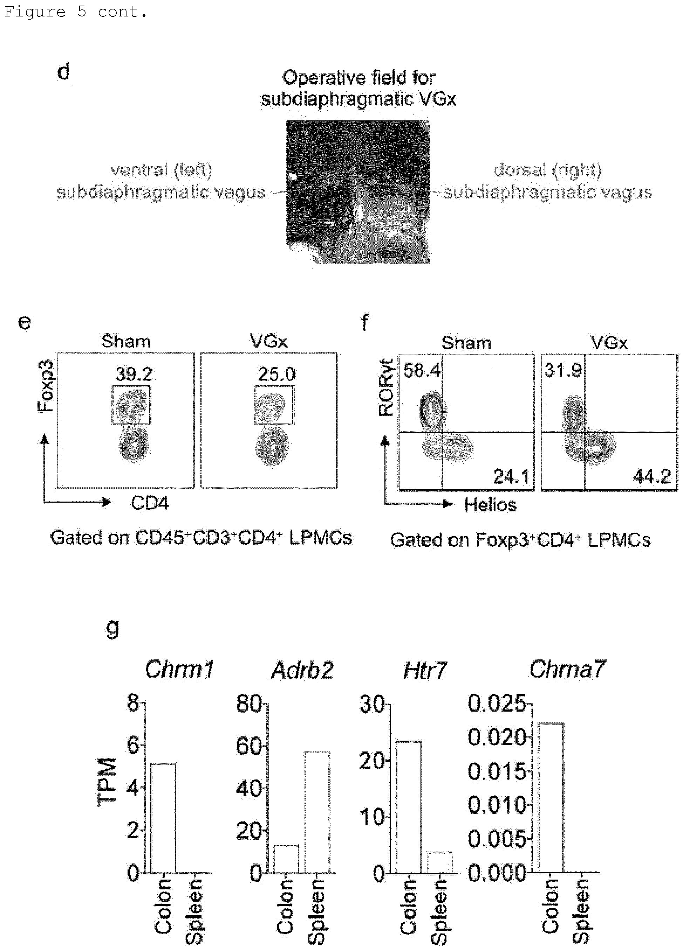

Indeed, the gastrointestinal (GI) tract is highly innervated as well as immensely populated by adaptive and innate immune cells ^{14,17}. Consistently, immunohistochemical analysis of the localization of neurons (β-tubulin III⁺) and MHC-II⁺ APCs, mainly consisting of CX3CR1⁺ mononuclear phagocytes (MNPs), revealed the close proximity of neurons and APCs in the colonic LP (Figures 1a, 1b, 5a, and 5b). Intestinal APCs, particularly CX3CR1⁺ MNPs and CD103⁺ dendritic cells (DCs), produce RA, which preferentially supports the development of pTregs in the TGF-β-rich microenvironment in the gut ^{8,18-23}. Despite the known immunoregulatory effects of the autonomic nervous system ^{1-3,15-17}, it remains incompletely understood how the vagus nerve influences gut homeostasis by regulating intestinal APCs and pTregs. To examine the immunological functions of the vagus nerve, the present inventor performed subdiaphragmatic truncal vagotomy (hereinafter, referred to as "VGx") in wild-type (WT) C57BL/6 (B6) mice (Figures 5c and 5d). The vagotomized mice showed a significant reduction in the number of Foxp3⁺ T helper cells, particularly Helios⁻RORγt⁺ pTregs, in the colon compared with the sham-operated mice (Figures 1c, 1d, 5e, and 5f). In addition to the reduction of colonic pTregs, there was a marked decrease in the levels of *Aldh1a1* and *Aldhla2,* encoding the RA-synthesizing enzymes RALDH1 and RALDH2, and in aldehyde dehydrogenase activity in colonic APCs (Figures 1e and 1f).

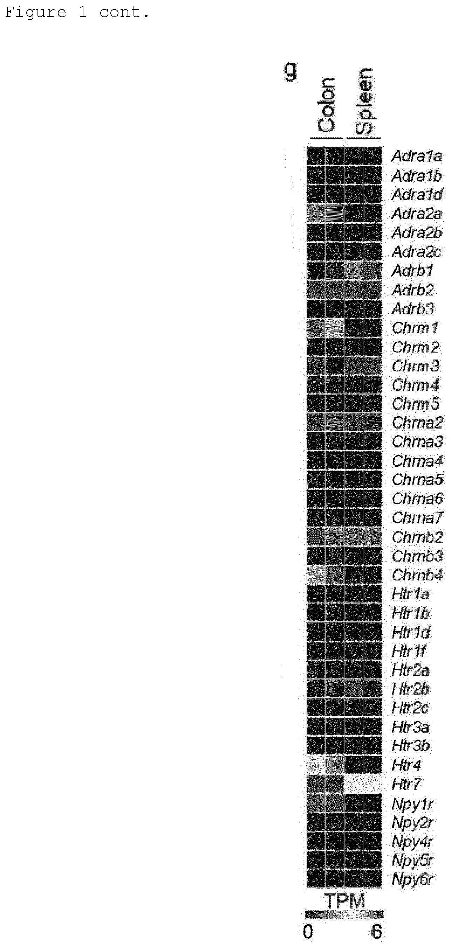

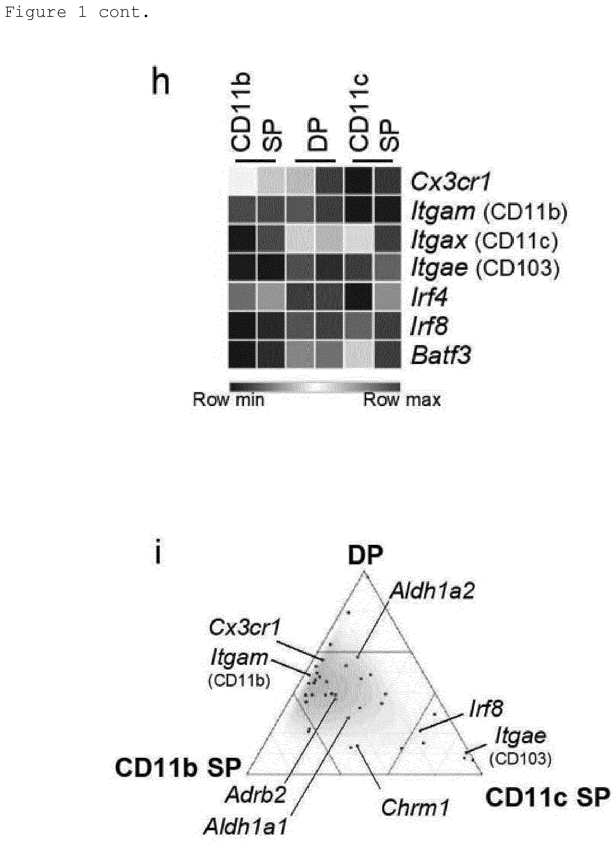

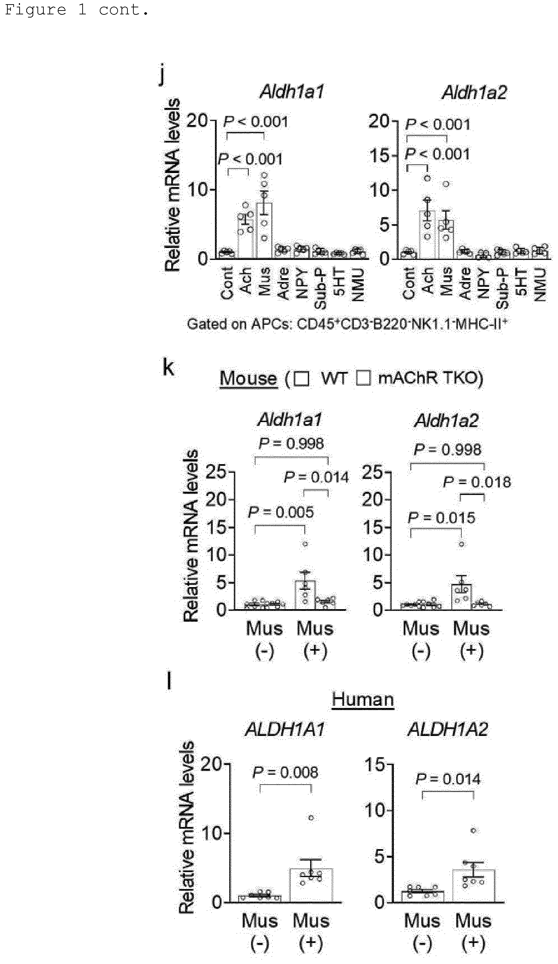

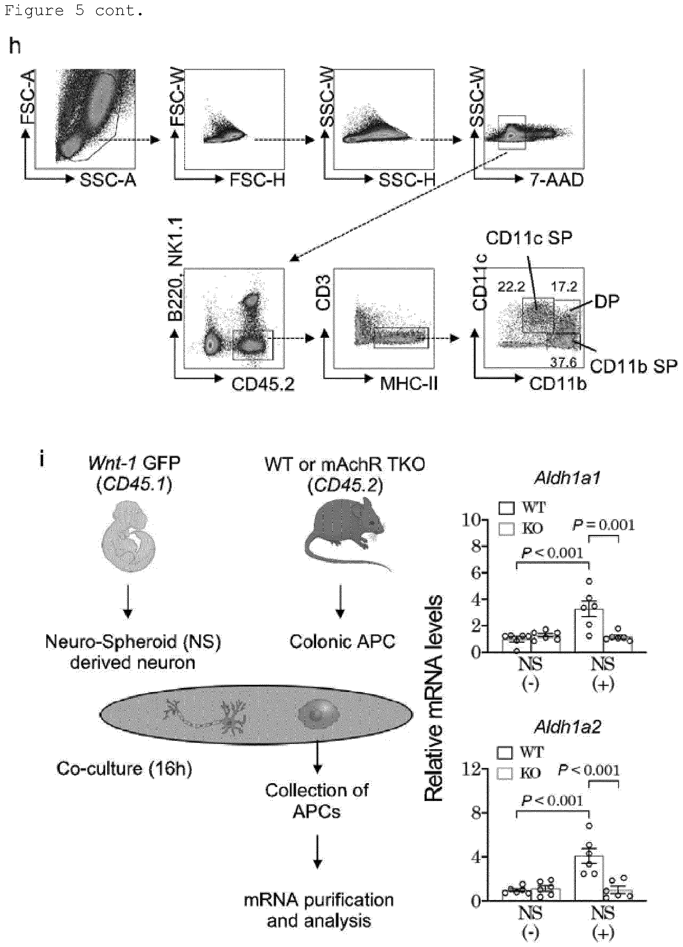

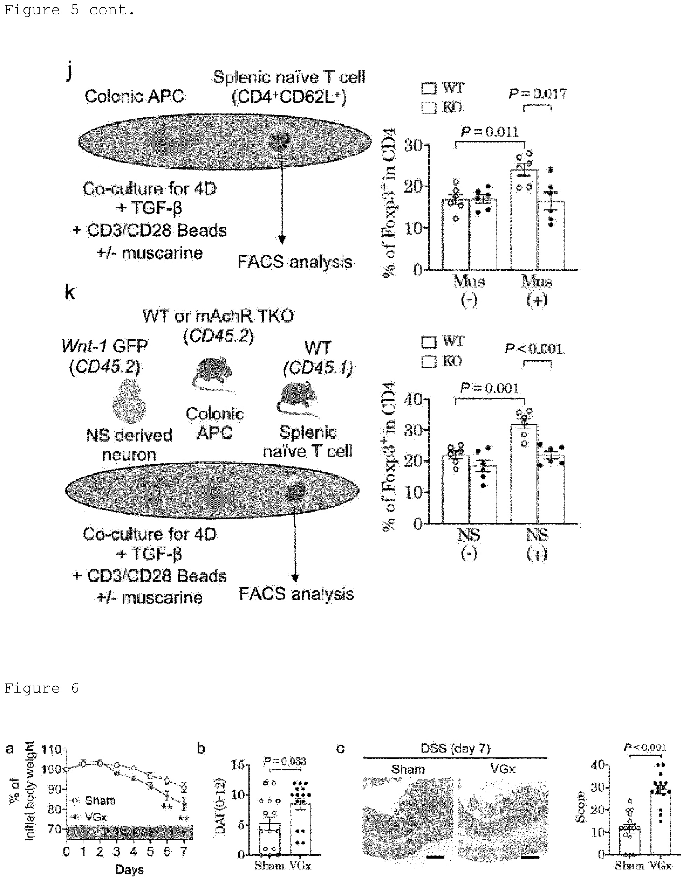

To identify the neurotransmitter responsible for conveying signals from the enteric neurons to colonic APCs, the present inventor performed mRNA-seq on APCs obtained from the spleen and intestine. The gut APCs exhibited higher levels of the gene encoding the muscarinic ACh receptor, *Chrm1*, than splenic APCs, suggesting a tissue-specific role for neurotransmitters in regulating intestinal APCs (Figures 1g and 5g). Of note, the expression of *Chrm1* as well as *Aldh1a1* and *Aldhla2* are shared within APC fractions enriched in CX3CR1⁺ MNPs and CD103⁺ DCs compared with the genes that define prototypical APC subsets, such as *Itgae* (CD103), *Cx3cr1,* and *Irf8* (Figures 1h, 1i, and 5h). The present inventor confirmed this finding by evaluating quantitative expression of *Aldh1a1* and *Aldhla2* on colonic APCs stimulated with multiple neurotransmitters, including Ach, muscarine, adrenaline, neuropeptide Y, substance P, serotonin (5-HT), and neuromedin U (Figure 1j). Furthermore, muscarine and enteric neuro-spheroids induced *Aldh1a1* and *Aldhla2* expression in colonic APCs obtained from WT mice and human intestine (Figures 1k and 1l), while co-culture of APCs deficient in *Chrm1, 2, and* 4 (mAChR TKO) with neuro-spheroids failed (Figures 5i and 5j). Accordingly, generation of Foxp3⁺ Tregs was enhanced by colonic APCs from WT mice, but not from mAChR TKO mice, precultured with either muscarine or neuro-spheroids (Figures 5k to 5n). Collectively, these results suggest that ACh-mAChR signaling in APCs contributes to harboring pTreg population in the gut.

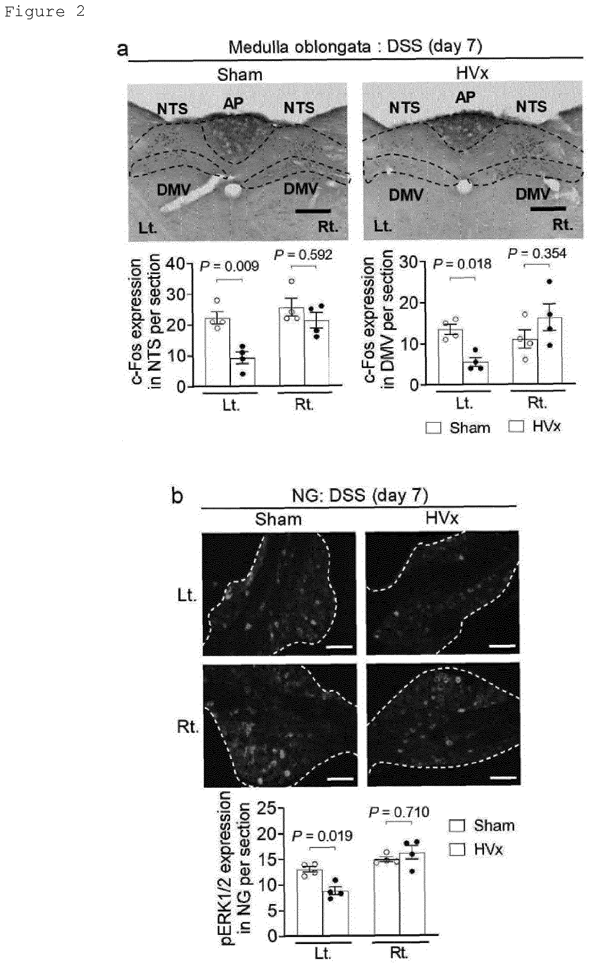

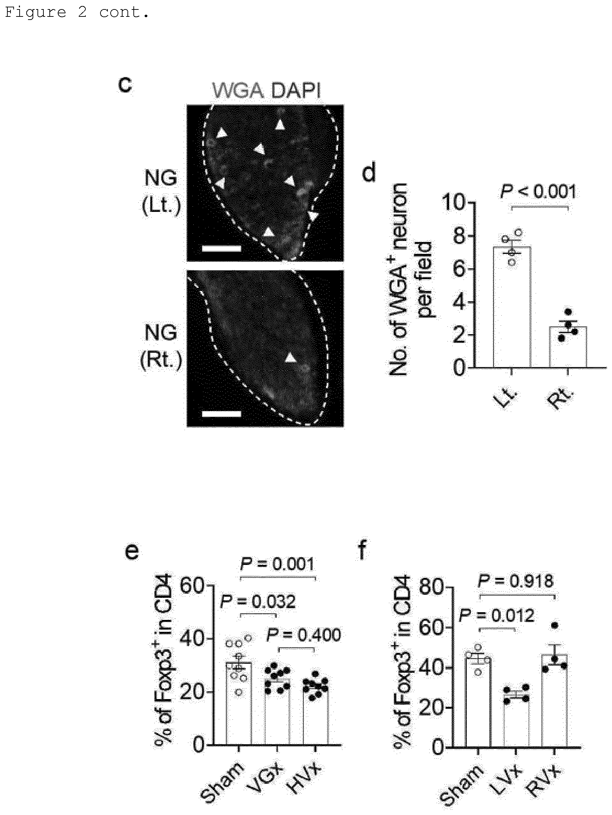

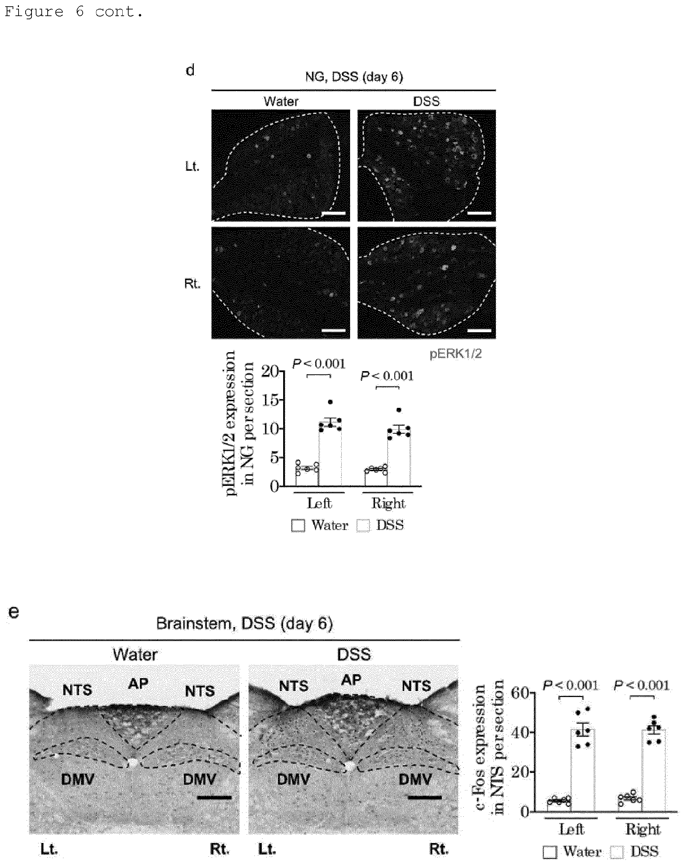

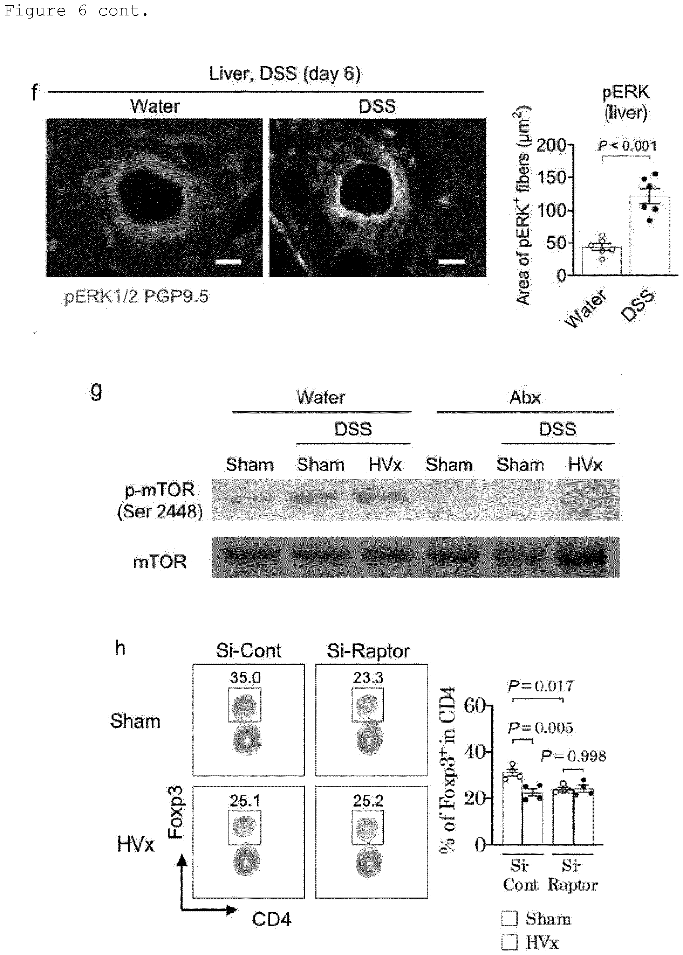

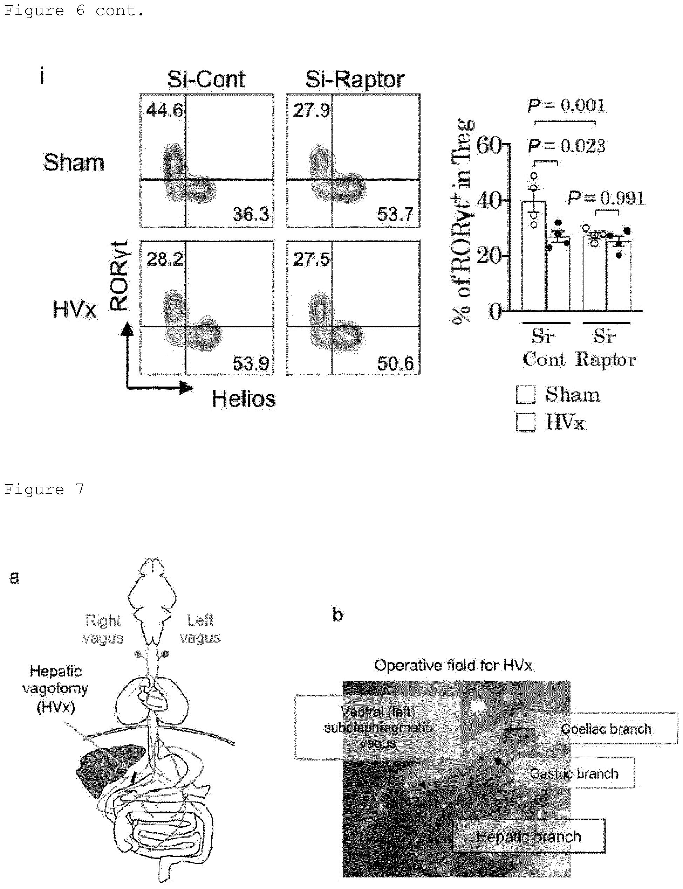

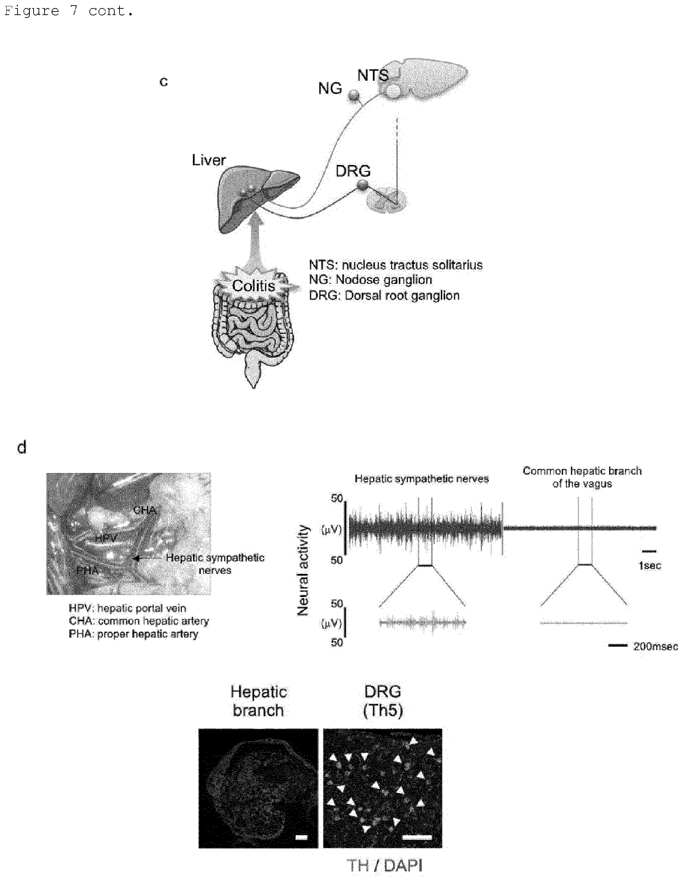

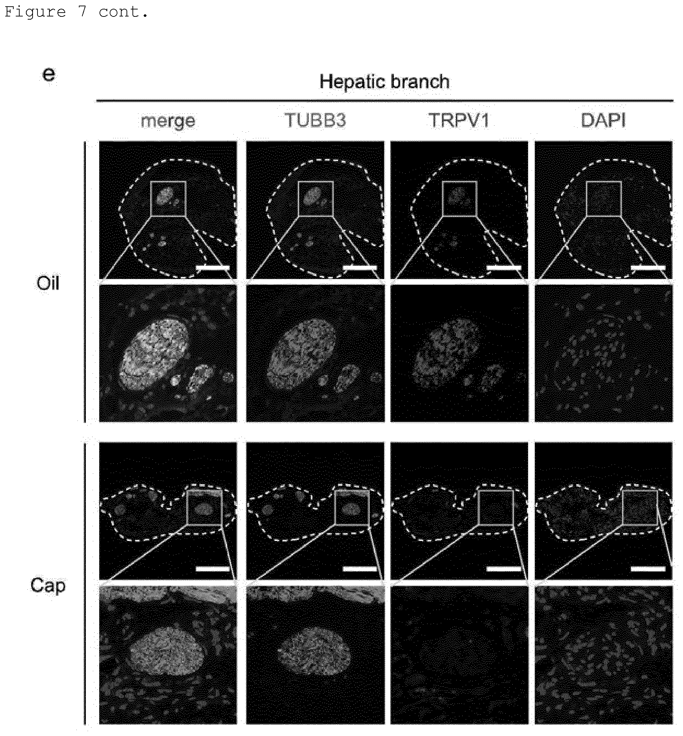

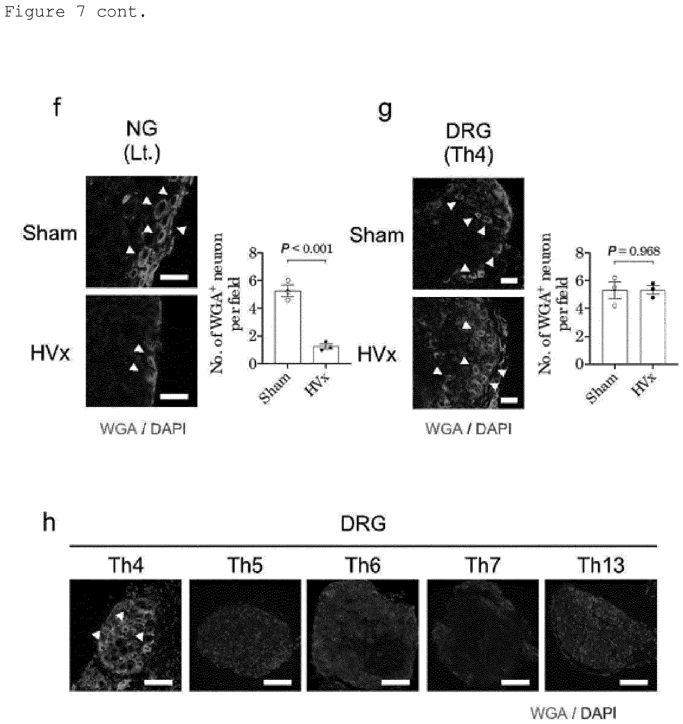

To explore this hypothesis, the present inventor assessed the requirement for signals via the vagus nerve in preventing intestinal inflammation. VGx resulted in increased susceptibility to dextran sulfate sodium (DSS)-induced colitis model (Figures 6a to 6c). Given that VGx reduced the number of pTregs and induced the local inflammatory environment, the present inventor next sought to determine which afferent neurons of the vagus nerve that are involved in the regulation and maintenance of the gut pTreg pool. The vagus nerve innervates a large part of the GI tract, and its afferent neurons relay sensory inputs to nodose ganglions (NGs) bilaterally ²⁴. During colitis, these sensory inputs are further projected to the nucleus tractus solitarius (NTS) of the brainstem (Figures 6d to 6f). Notably, development of acute colitis led to activation of hepatic sensory afferents *in vivo,* which was abrogated by the selective surgical division of the common hepatic branch of the vagus nerve (hereinafter, referred to as "HVx" ²⁵) (Figures 2a, 2b, and 7a to 7c). As the liver is continuously exposed to nutrients, bacterial products, toxins, and metabolites from intestine, this gut-liver axis, connected by the portal circulation, has been demonstrated to contribute to liver diseases ^{26,27}. In addition, nutrients and bacterial products can activate the vagus nerve via mechanistic target of rapamycin complex 1 (mTORC1) signaling ²⁸ (Figures 6g to 6i), suggesting that the hepatic sensory afferents of the vagus nerve are activated during colitis. Indeed, hepatic retrograde tracing supported the ideas that the liver senses the gut microenvironment, activates the hepatic sensory afferents of the vagus nerve, and transmits the signals to the brain via the left NG (Figures 2c and 2d). Importantly, the common hepatic branch of the vagus nerve that is divided in HVx mice predominantly consists of capsaicin-sensitive TRPV1⁺ sensory afferents without sympathetic TH⁺ neurons, as determined by electrophysiological and immunohistological assessment (Figures 7d and 7e). Capsaicin deafferentation of the common hepatic branch of the vagus nerve significantly reduced the number of pERK positive cells in the left, but not the right, NG (Figure 2b). In addition, the number of retrogradely-labelled cells was significantly diminished in the left NG, but not in the DRG in HVx mice, indicating that the common hepatic branch of the vagus nerve sends signals through the left NG, but not the right NG or the DRGs (Figures 2c and 7f to 7h). These results suggest that sensory information of intestinal environment is transmitted to the brain via lateralized ascending pathways of the left vagus nerve from the liver to the brain.

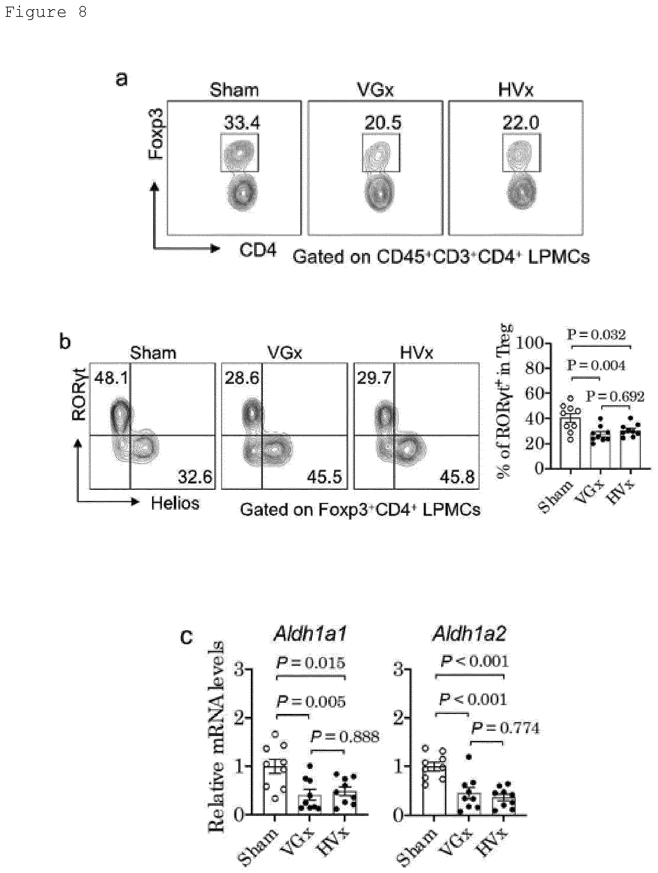

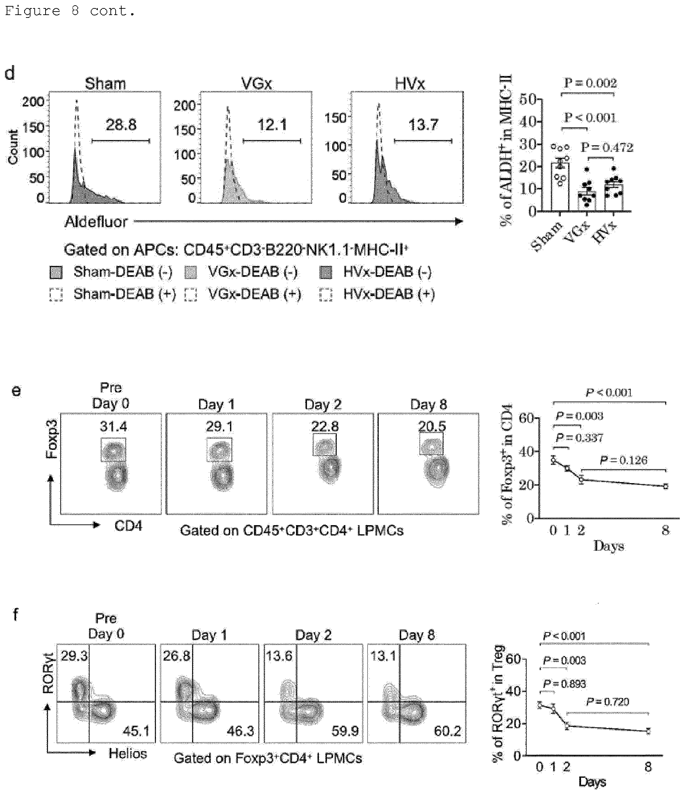

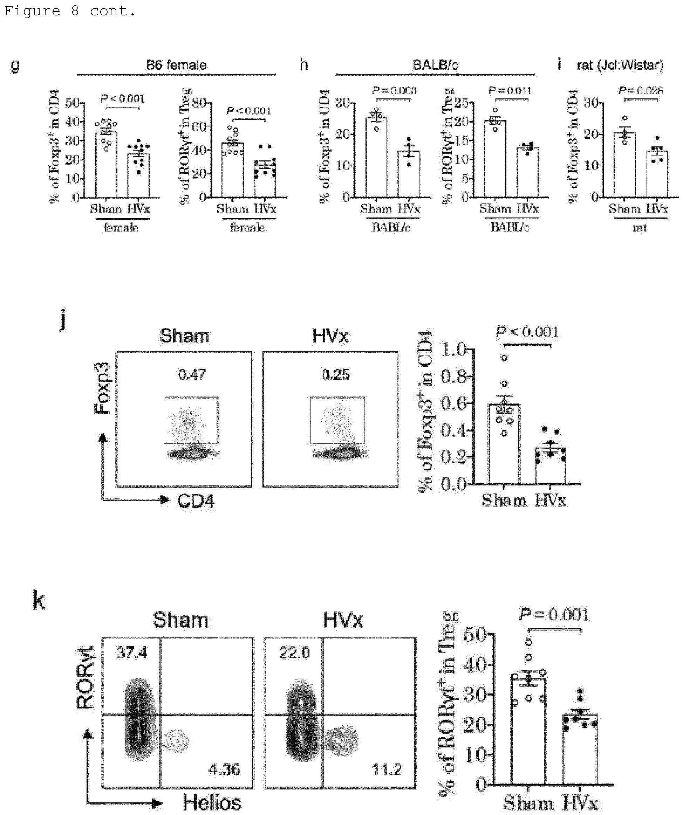

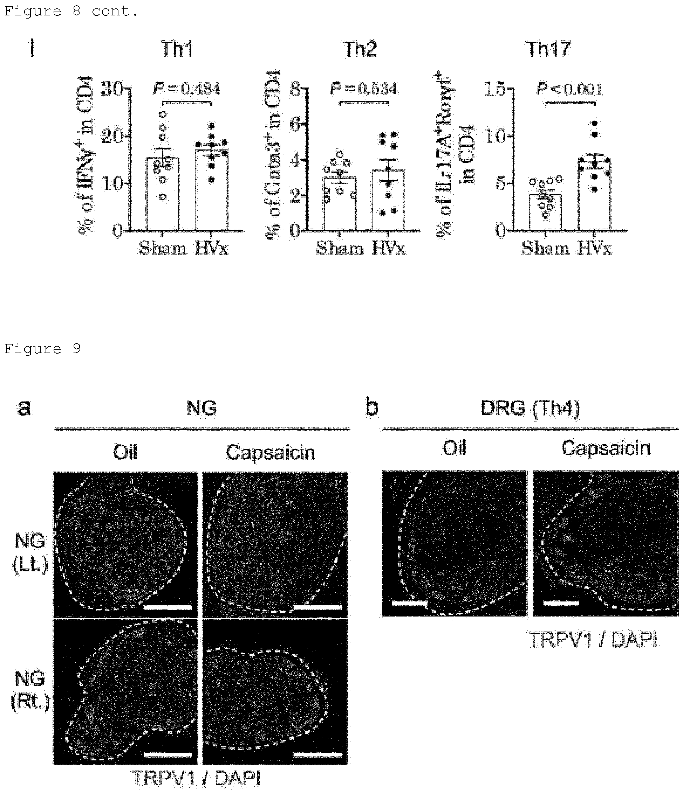

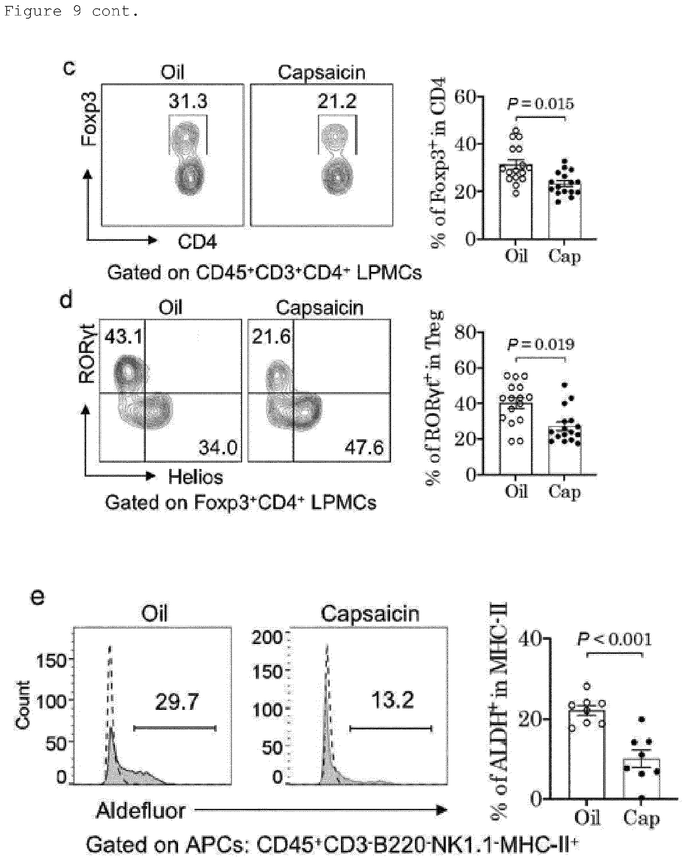

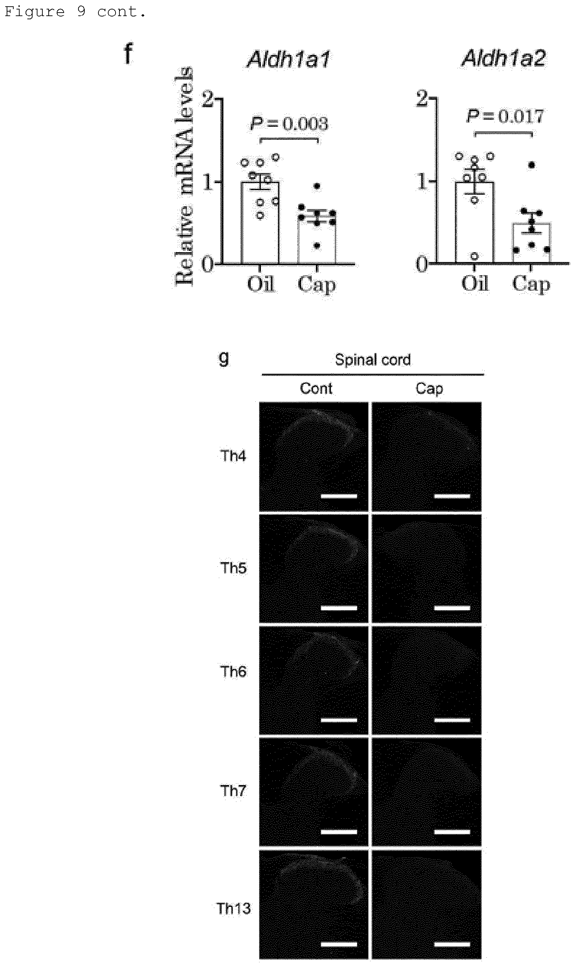

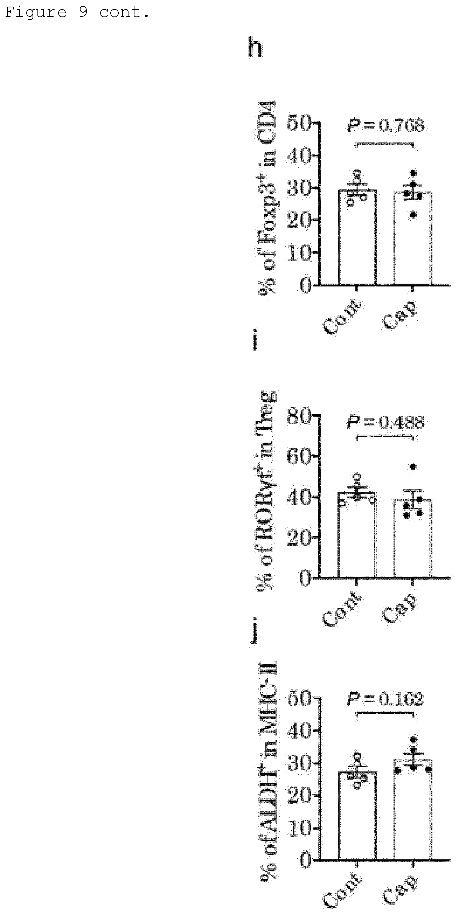

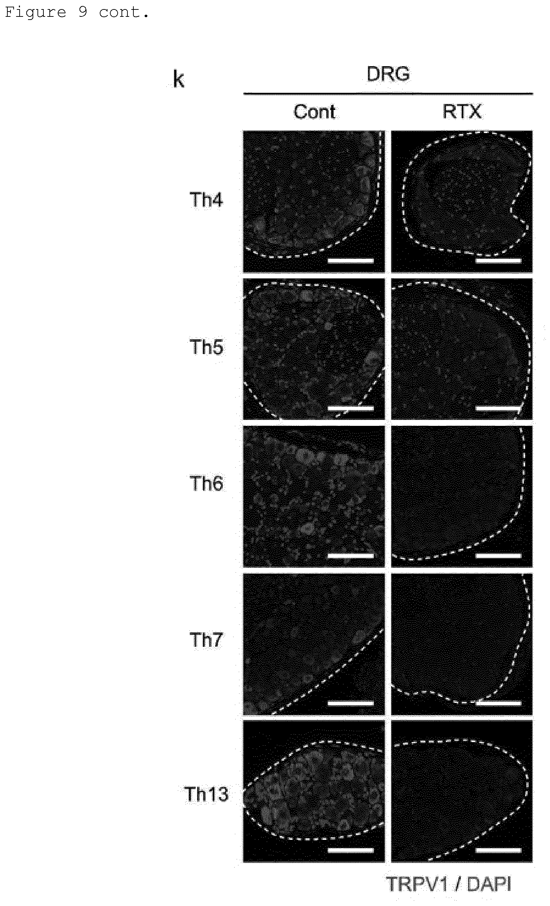

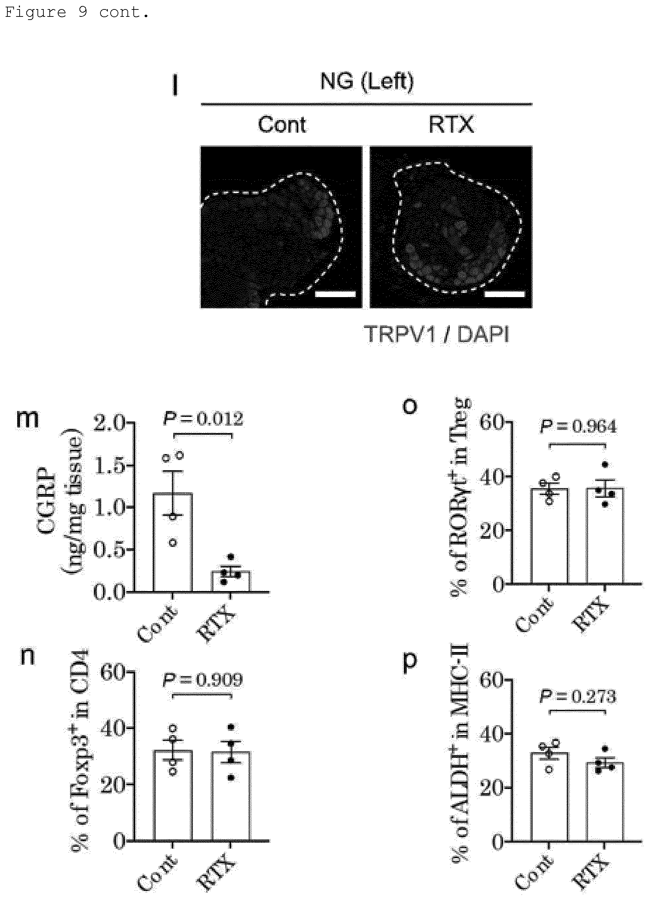

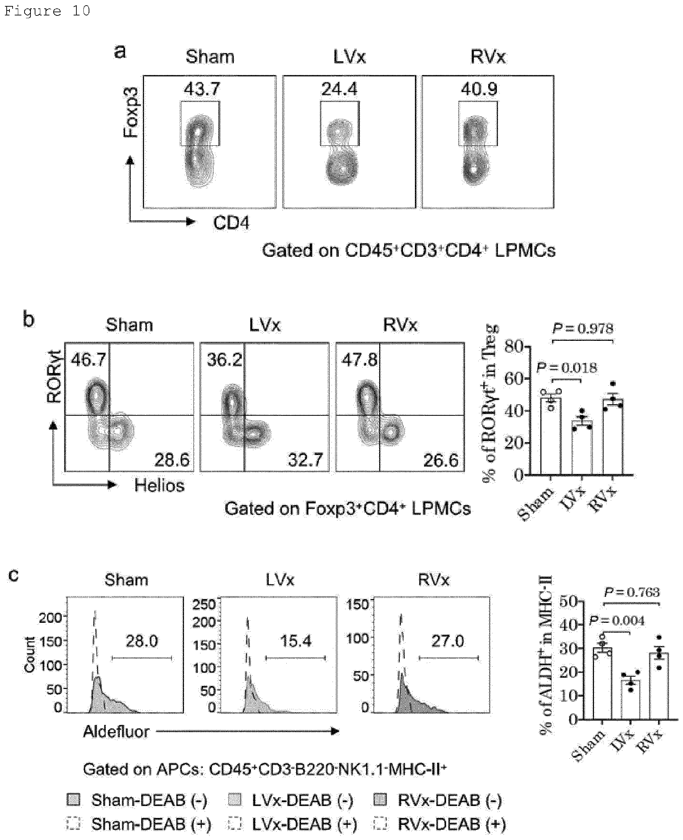

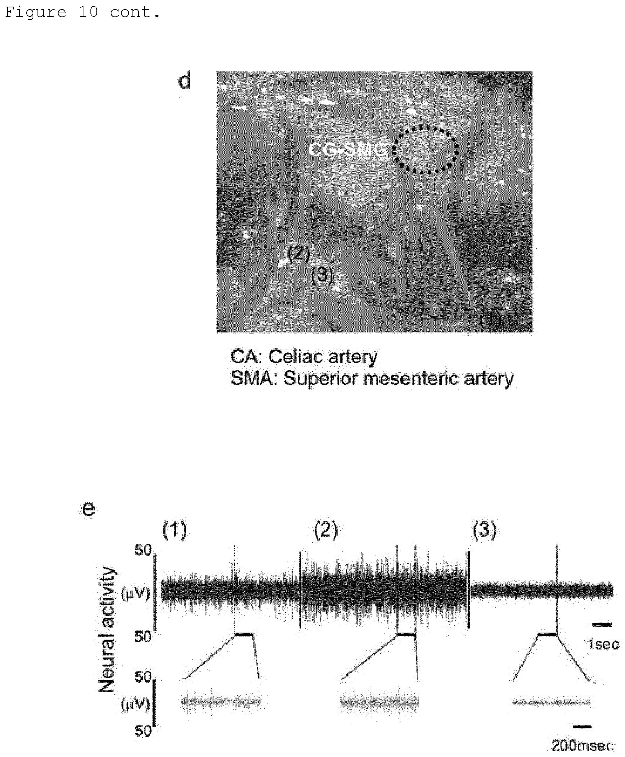

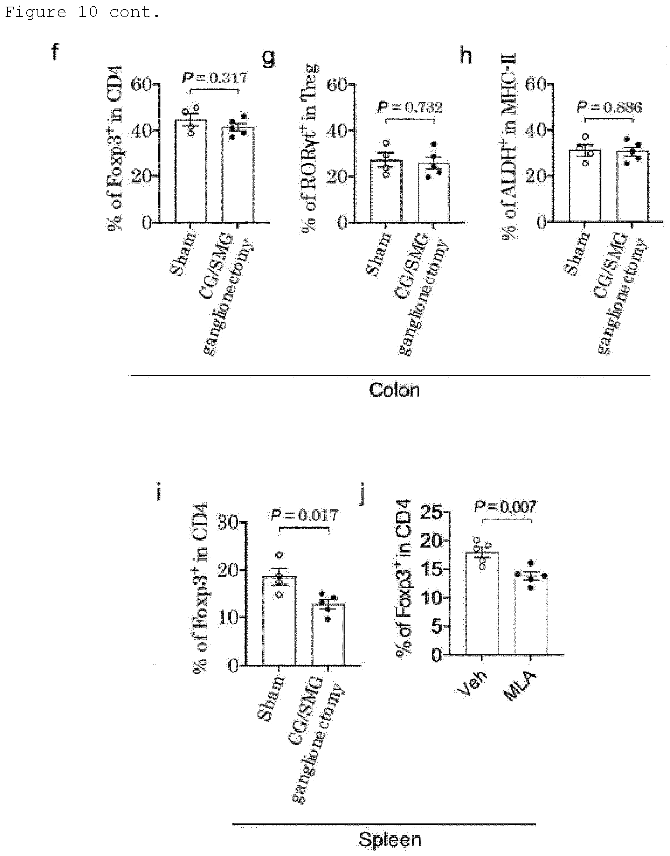

The anatomical lateralization of the vagus nerve led the present inventor to explore how the hepatic vagal sensory afferents impacts on the gut Tregs, and the present inventor characterized the effects of HVx on the gut and the spleen. As a result, the present inventor observed a significant reduction in the proportion of pTregs among CD4⁺ T cells as well as the expression and activity of aldehyde dehydrogenase in APCs obtained from the large intestine of HVx and VGx mice compared with the sham-operated mice (Figures 2e and 8a to 8d). This HVx-induced decrease in colonic pTregs occurred rapidly at day 2 and consistently across rodent sexes, strains and species (Figures 8e to 8i). Since *in vivo* generated pTregs show demethylated Treg-specific demethylation regions (TSDRs), the rapid reduction in pTregs in HVx mice could be attributed to the epigenetic effects of the vagus nerve on maintenance and stability of gut pTregs by changing the DNA methylation status at TSDRs ²⁹⁻³⁴. Consistently, HVx impaired pTreg differentiation and stability in T cell reconstituted mice and unleashed the RA-mediated repression of the Th17 differentiation program (Figures 8j to 81). The importance of hepatic sensory afferents in the left NG on maintaining proper reservoir of gut Tregs was further supported by the finding that perturbation of hepatic vagal sensory afferents to left NG by perivagal capsaicin treatment, but not deafferentation of DRGs by intrathecal capsaicin or resiniferatoxin (RTX) treatment, resulted in reduced colonic Treg numbers and aldehyde dehydrogenase activity in APCs (Figure 9). This suggests the functional asymmetry in the vagal afferents (Figures 2f and 10a to 10c). Maintenance of colonic Tregs is less dependent on the sympathetic nervous system than that of MMs and ILC2s as previously reported in the context of viral and parasite infection ^{35,36} (Figures 10d to 10h). Contrary to the effects in the small and large intestine, HVx mice exhibited normal frequencies of splenic Tregs, while surgical ablation of CG-SMG and chemical blockade of the β2 adrenergic receptor or the 7-nicotinic Ach receptor (7-nAchR) showed a significant reduction in splenic Tregs (Figures 10i to 10o). Since the splenic nerve mainly comprised of adrenergic fibres arising from CG-SMG has previously been reported to suppress T cell activation and inhibit systemic cytokine production from the splenic macrophages through 7-nAchR ³⁷⁻⁴², ablation of CG-SMG or the splenic nerve rather than vagotomy itself was predicted to affect the splenic Treg population. These results support that the liver-brain-gut neural arc monitors the gut microenvironment and tunes the levels of gut pTregs by sending Ach signaling and controlling colonic APCs.

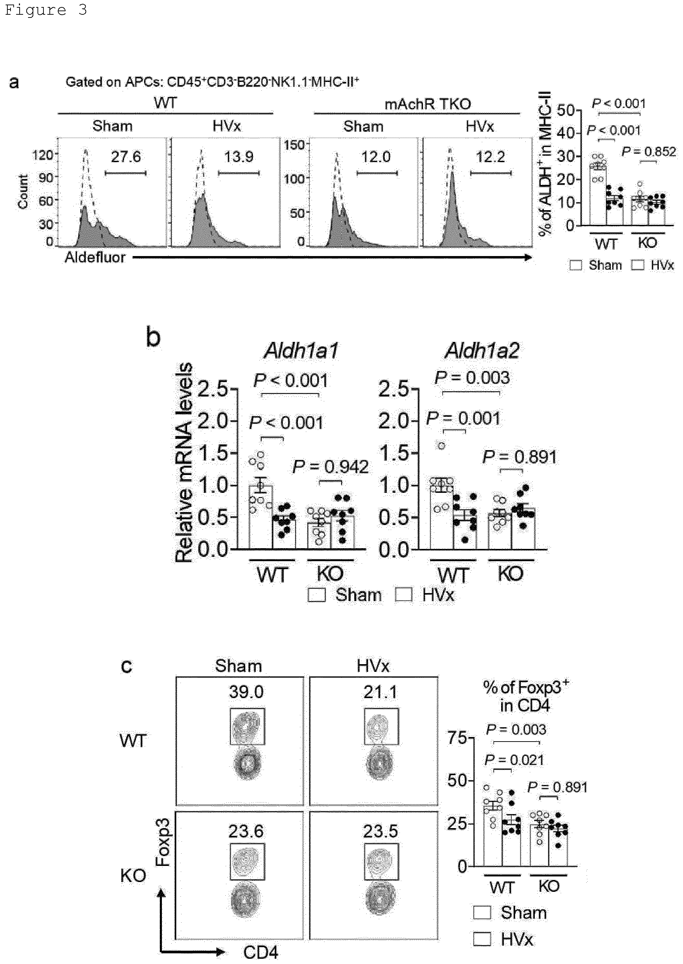

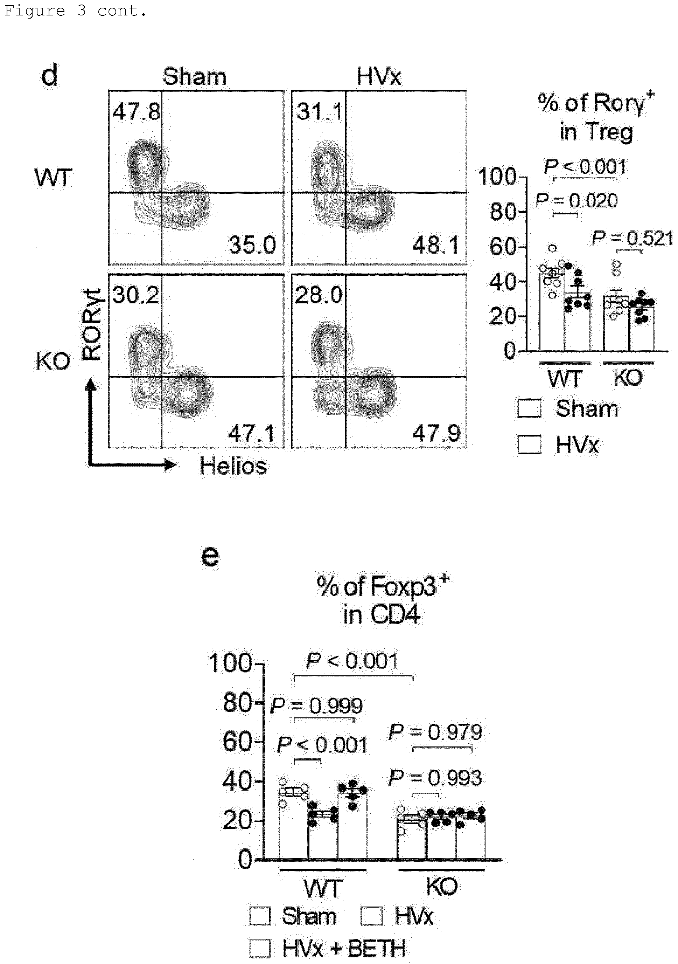

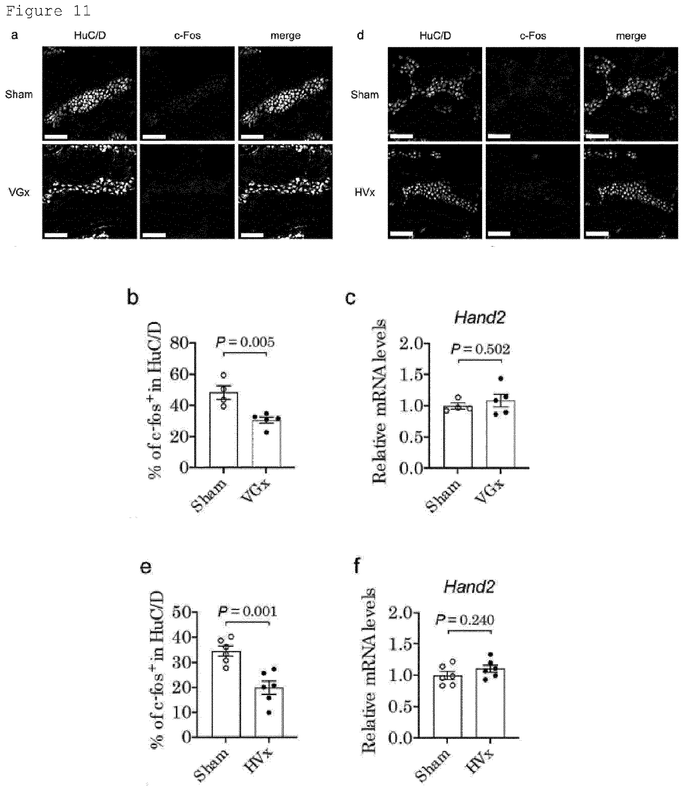

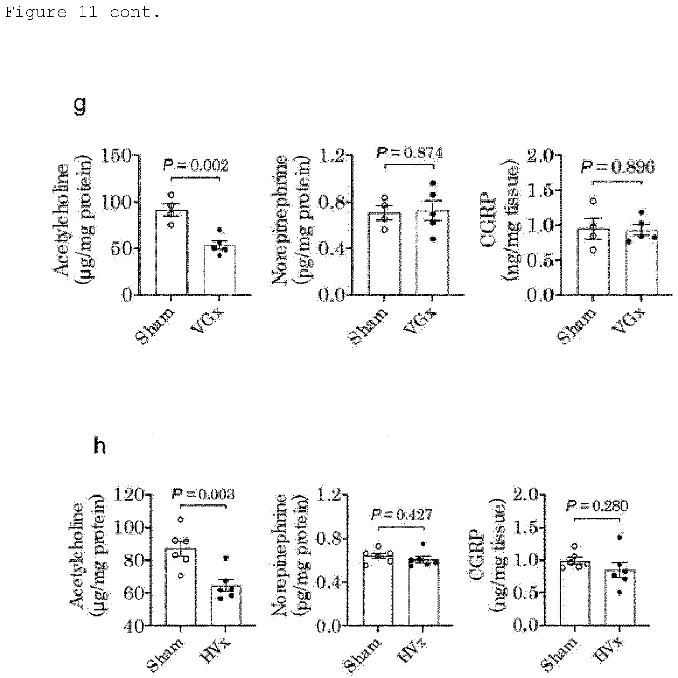

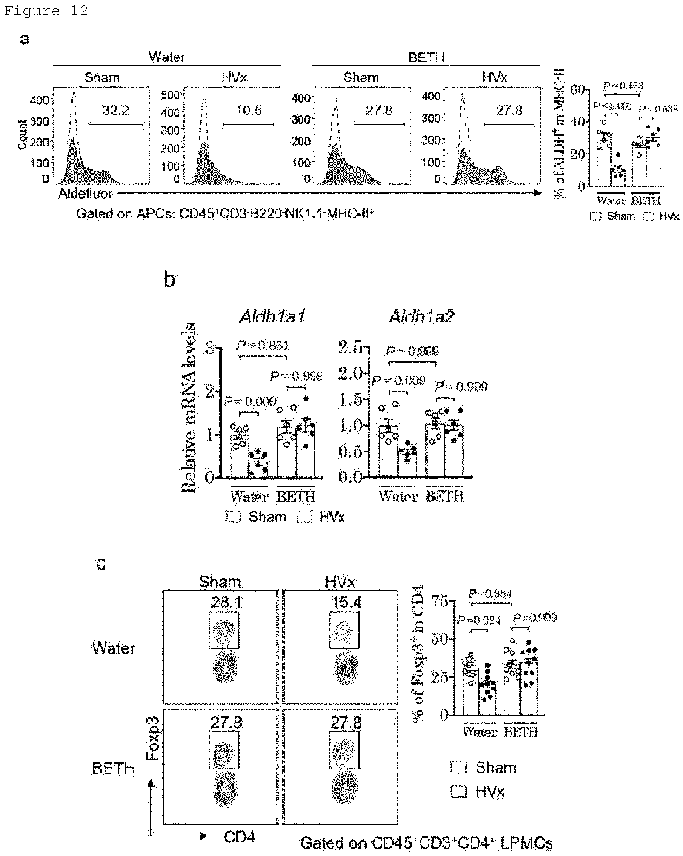

This prompted the present inventor to investigate the role of muscarinic Ach signaling in intestinal APCs *in vivo.* Genetic ablation of mAChRs was associated with reduced *Aldh1a1* and *Aldhla2* expression in colonic APCs, resulting in the reduced pTregs in the colon (Figures 3a to 3d). In VGx and HVx mice, the present inventor observed fewer c-Fos⁺ enteric neurons in the colonic myenteric plexus compared with sham-operated mice, while the expression of *Hand2,* a transcription factor required for terminal differentiation of enteric neurons, was not affected (Figures 11a to 11f). In addition, intestinal small-molecule and peptide neurotransmitters for parasympathetic system (Ach), but not sympathetic (noradrenaline) and sensory (calcitonin gene-related peptide (CGRP)) systems, was decreased in VGx and HVx mice compared to that in sham-operated mice (Figures 11g to 11h). Given that hepatic-selective and truncal vagotomy primarily reduced local Ach levels in the gut, the present inventor tested whether mAChR activation could restore aldehyde dehydrogenase expression and activity in the gut APCs. Treatment with an mAChR agonist, bethanechol, restored *Aldh1a1* and *Aldhla2* expression in colonic APCs in HVx mice compared with sham-operated mice, while a 7-nAchR agonist and antagonist had minimal contribution (Figures 12a to 12j) just like genetic deletion of 7-nAchR ⁴³. Consistently, these HVx WT mice, but not in mAChR TKO mice, treated with bethanechol exhibited an increased frequency of pTregs, suggesting that the liver-brain-gut neural arc stimulates colonic APCs and creates the pTreg niche (Figures 3e, 12k, and 121). Collectively, these results support that the neural input from the hepatic sensory afferents is essential for initiating this vago-vagal liver-brain-gut neural arc and this reflex arc is independent of sympathetic system and axon reflex.

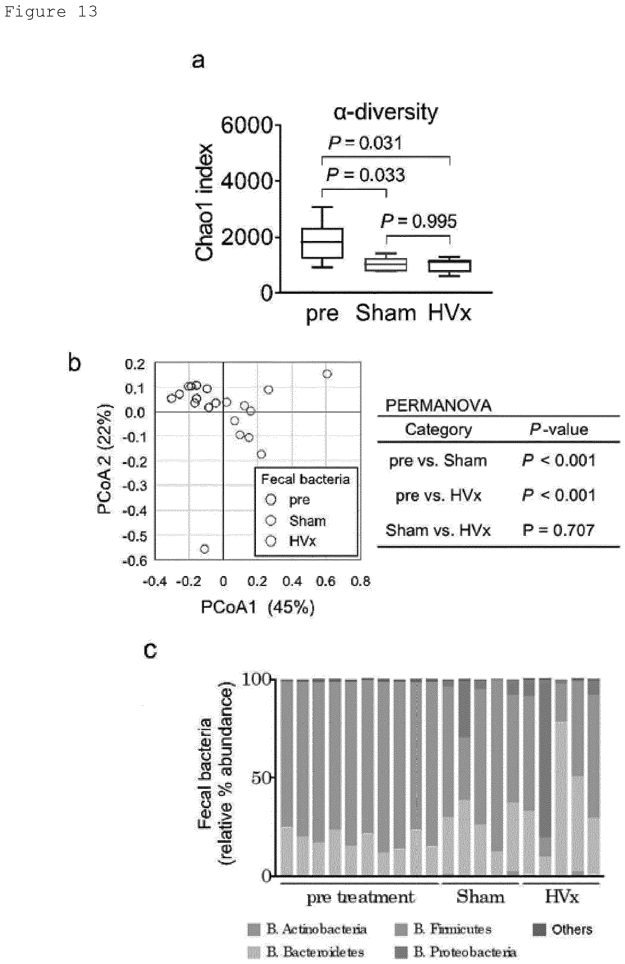

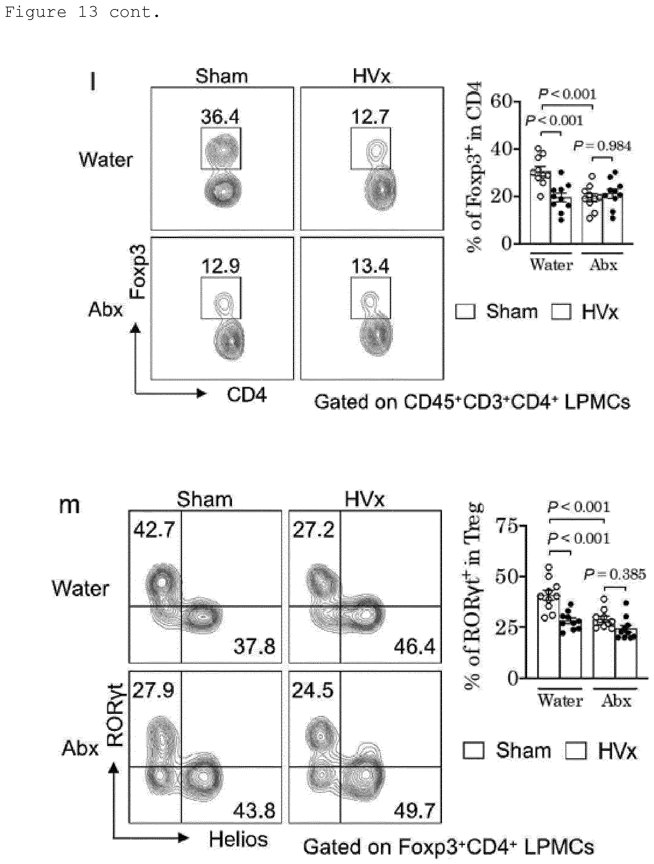

As pTreg generation and maintenance is highly dependent on the microbiome and metabolites, the present inventor investigated the role of the microbiome in pTreg generation and maintenance. The gut microbiome derived from HVx mice and the sham-operated control mice showed no significant differences in composition and diversity, and transferring fecal bacteria from these mice induced comparable amount of gut pTregs in germ-free mice (Figures 3 and 13a to 13e). This suggests that the liver-brain-gut neural arc functions to harbor a pTreg pool independently of HVx-induced alterations in gut microbiome and metabolites. In addition, the present inventor observed no further reduction in pTregs, particularly microbiome-independent pTregs ¹³, in gut-sterilized HVx mice (Figures 13l and 13m). Collectively, these data indicate that the liver-brain-gut neural arc maintain basal levels of gut pTregs which are dependent on tonic microbial input.

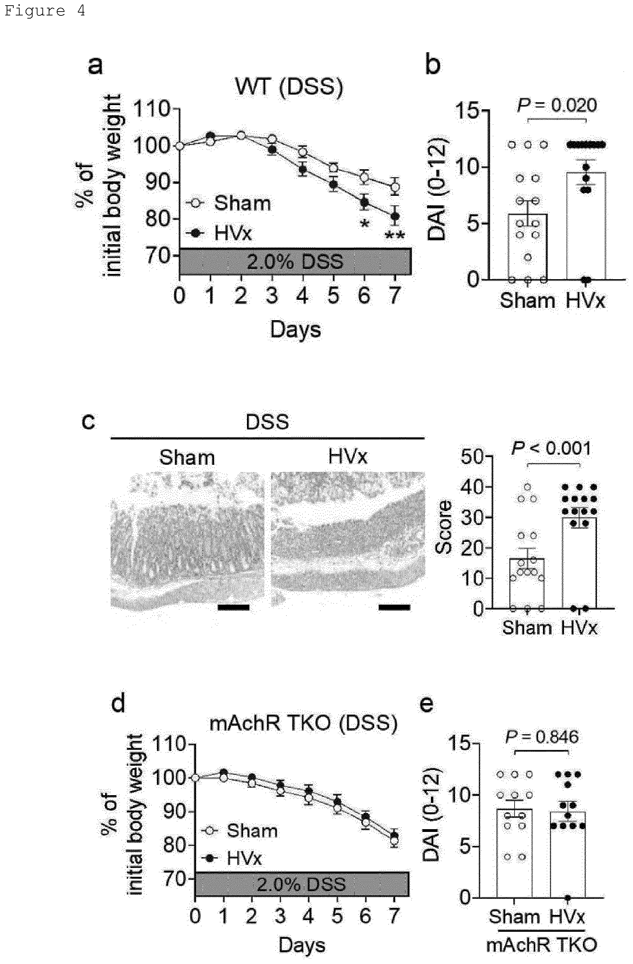

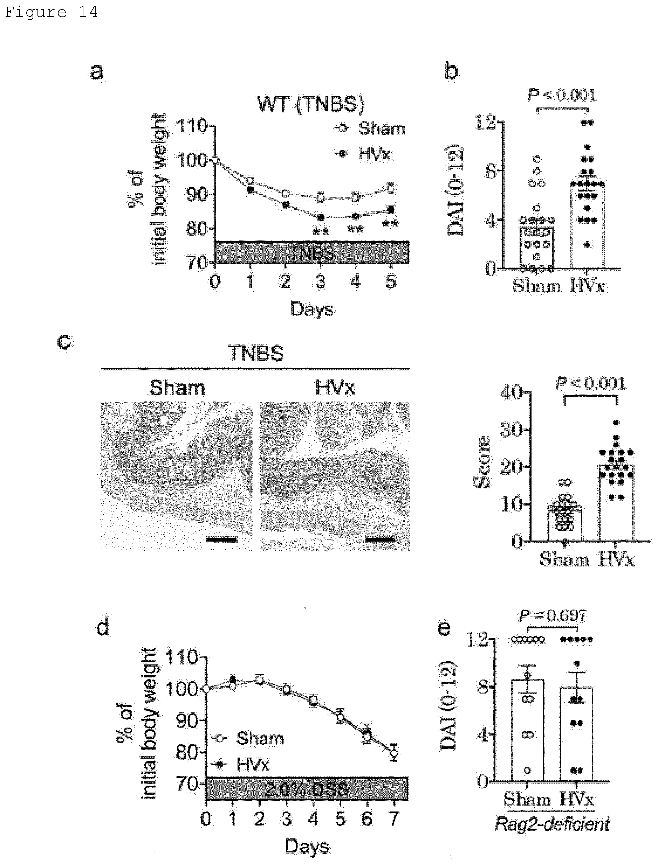

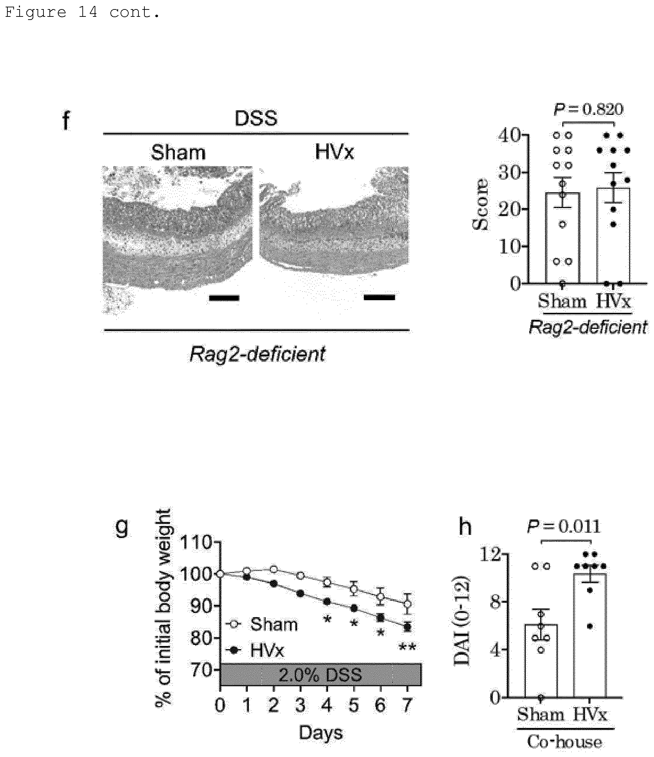

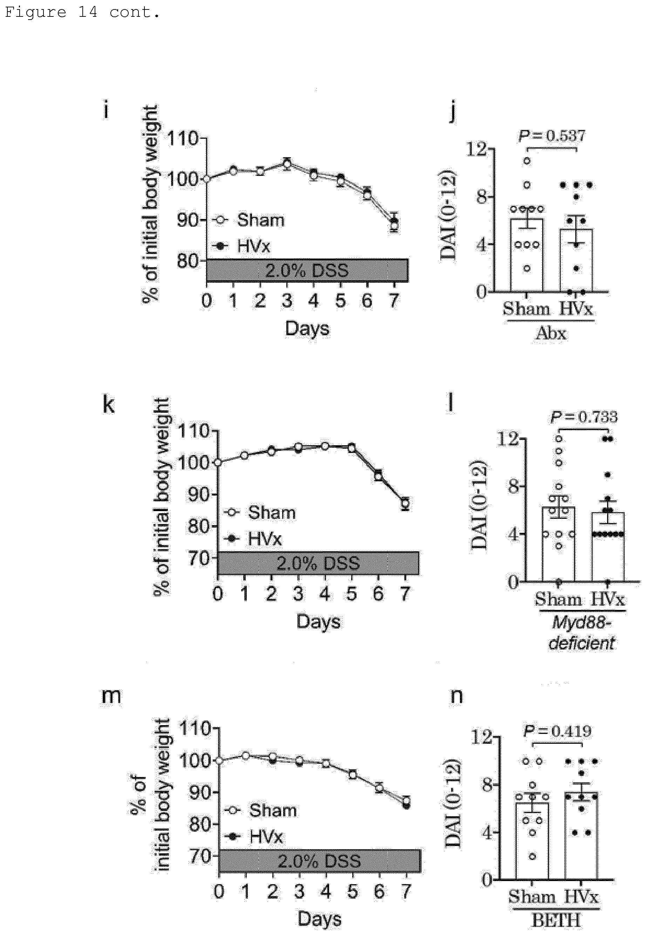

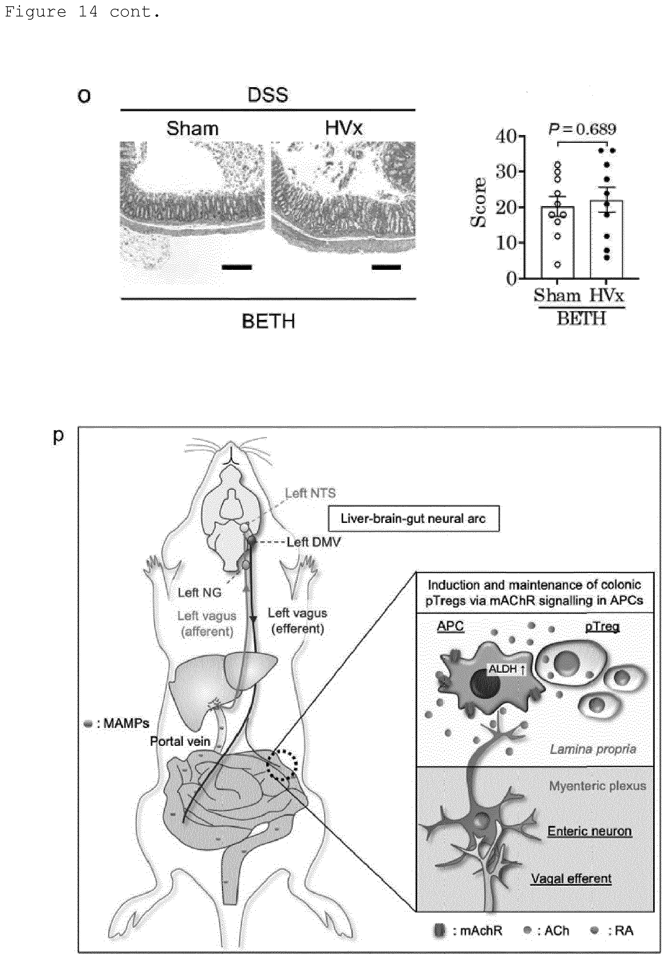

This action of the liver-brain-gut neural arc as a modulator of intestinal pTregs was previously unanticipated, so the present inventor sought to determine if it is relevant to the development of colitis. Mice treated with surgical or chemical division of the hepatic vagal branch exhibited reduced pTreg frequencies (Figures 3a, 8a, 8b, 5c, and 5d), which resulted in the increased susceptibility to colitis induced by DSS and 2,4,6-trinitrobenzene sulfonic acid (TNBS) (Figures 4a to 4c and 14a to 14c). Consistently, *Rag2*^{-/-} mice did not show worsened colitis by HVx unlike T-cell-insufficient mice (Figures 14d to 14f). In addition, splenectomy had little effect on the severity of colitis in the HVx mice, unlike in endotoxemia models ³⁷⁻³⁹ (data not shown). As HVx did not lead to significant changes in gut microbiome composition (Figures 13a to 13c), HVx mice exhibited severer colitis than co-housed sham-operated mice (Figures 14g and 14h). In addition, neither antibiotic-treated mice nor MyD88-deficient mice exhibited increased susceptibility to DSS-induced colitis by HVx (Figures 14i to 14l), suggesting that persistent microbial input is required for functioning the liver-brain-gut neural arc to harbor gut pTreg pool. By contrast, exacerbation of DSS-induced colitis in the HVx mice was inhibited by a cholinergic agonist (Figures 4g to 4i and 14m to 14o ). Collectively, these data indicate that the liver-brain-gut neural arc serves as a feedback loop to protect the intestine from an excessive inflammation (Figure 14p).

In summary, this work reveals an intriguing activity of extrinsic vago-vagal reflexes that connects hepatic vagal sensory afferents, the brainstem, and the vagal efferents, and the enteric neurons to stimulate mAChR⁺ APCs and maintain a reservoir of peripheral regulatory T cells. As demonstrated by a recent retrospective cohort study that reported increased risk of patients with new-onset depression developing IBDs ⁴⁴, autonomic imbalance is likely to contributes to the pathogenesis of IBD. In addition to the direct and mutual gut-brain neural reflexes that control appetite, food reward, cancer, fatty liver, Parkinson's disease, and other neural diseases ⁴⁵⁻⁴⁸, the findings of the present inventor provide a unique view of tissue-specific immune cell adaptation mediated by both the liver and CNS. Dysfunction of this liver-brain-gut neural arc predisposes the gut to inflammation, raising the possibility that denervation-induced suppression of tumorigenesis could be attributable to the decreased number of colonic pTregs. This work highlights the essential roles of the liver-brain-gut neural arc that specifies the immunoregulatory niche and fine-tunes immune responses in the intestine. Interventions that target this liver-brain-gut neural arc could provide broad applications to promote the treatment of IBD ⁴⁹, infectious diseases, and cancer of the gut.

### [Experimental methods]

### Animal

C57BL/6 (*WT*) mice, BALB/c mice and Jcl:Wistar rats were purchased from Japan CLEA (Tokyo, Japan). 5-week-old male germ free (GF) mice (C57BL/6 background strain) were purchased from Sankyo Lab Service Corporation and were kept in the GF Facility of Keio University School of Medicine. Ly5.1 mice, Foxp3^{CreERT2} mice, *Cx3cr1*^{*GFP*/*GFP*} transgenic (*Cx3cr1^{gfp}*) mice, *Rag2* knockout (*Rag2*^{-/-}) mice and *Myd88* knockout (*Myd88*^{*-*/*-*}) mice were obtained from The Jackson Laboratory (Maine, USA). *Chrm1*/*Chrm2*/*Chrm4* triple-knockout (mAChR TKO) mice were obtained from Center for animal resources and development (Kumamoto, Japan). Wnt1 promoter/enhancer (Wnt1-Cre) were mated with EGFP reporter mice (CAG-CATloxP/loxP-EGFP) to obtain Wnt1-Cre/Floxed-EGFP double-transgenic mice ⁵⁰.
Foxp3^{CreERT2} mice were mated with floxed-tdTomato reporter mice ⁵¹ to obtain Foxp3-reporter mice. Mice at the age of 6 to 8 weeks were used in all experiments. All mice were maintained under SPF conditions in the Animal Care Facility of Keio University School of Medicine. All experiments were approved by the regional animal study committees (Keio University, Tokyo, Japan) and were performed according to institutional guidelines and Home Office regulations.

### Subdiaphragmatic and hepatic-selective vagotomy

Bilateral or unilateral (left or right) subdiaphragmatic vagotomy was performed as previously reported (Figures 5c and 5d) ⁵². A midline incision was made to provide wide exposure of the upper abdominal organ in male mice anesthetized with the combination of medetomidine, midazolam, and butorphanol. The bilateral subdiaphragmatic trunks of vagal nerves along the esophagus were exposed and cut. In the sham operation group, these vagal trunks were exposed but not cut. Selective hepatic vagotomy (HVx) was performed as described (Figure 7) ²⁵. The ventral subdiaphragmatic vagal trunk was exposed as described above under anesthesia. Since the common hepatic branch of the vagus forms a neurovascular bundle, this branch was selectively ligated by silk sutures and cut using microscissors. In the sham operation group, the common hepatic branch was exposed but not cut.

### Selective hepatic vagal afferent blockade by perivagal application of capsaicin

The hepatic branch of the vagal trunk was freed from the surrounding tissues by paraffin paper and then wrapped for 30 minutes with cotton bud soaked with vehicle (Tween 80: olive oil = 1:9) alone or 10 mg/ml capsaicin dissolved in the vehicle solution. The cotton string was removed 30 minutes later and the abdominal incision was closed ²⁸.

### Celiac and superior-mesenteric ganglionectomy

A midline incision was made to provide wide exposure of the upper abdominal organ in mice anesthetized with isoflurane. The celiac ganglia (CG) is attached to the superior mesenteric ganglion (SMG) via short nerve trunks (Figure 10d). The celiac and superior-mesenteric ganglion (CG-SMG) complex along the superior mesenteric artery was exposed and removed. In the sham operation group, the superior mesenteric artery was exposed but not remove ⁵³.

### Intrathecal administration of resiniferatoxin (RTX) and capsaicin

For targeted ablation of TRPV1⁺ neurons in DRG or spinal cord, mice were injected intrathecally with resiniferatoxin (RTX) (25 ng/mouse, vehicle; 0.25% DMSO / 0.02% Tween-80 / 0.05% ascorbic acid in PBS) or capsaicin (10 µg/mouse, vehicle; 10% EtOH / 10% Tween 80 in PBS) by using a 25 l Hamilton syringe with a 28 gauge needle. Control mice were injected with vehicle only. Phenotypes of colonic immune cells were analyzed at day 7 after injection. Depletion of TRPV1⁺ neurons in the DRG and spinal cord was confirmed by immunostaining.

### Parabiosis

The present inventor carried out parabiosis surgery as previously describe ⁵⁴. After shaving the corresponding lateral aspects of each mouse, matching skin incisions were made from the base of the anterior to posterior extremities of each mouse, and the subcutaneous fascia was bluntly dissected to create about 1/2 cm of free skin. The parabionts were then combined by suturing the corresponding free skin densely with surgical clips. At 2 weeks after the surgery, mice were subjected to sham or HVx.

### T cell reconstitution model

T cell reconstitution model was done as described previously ⁵⁴. Briefly, *Rag2*^{*-*/*-*} mice were injected intraperitoneally with 3 × 10⁵ FACS-sorted wild-type naive CD4⁺CD45Rb^{hi} cells. Mice were monitored weekly for body weight. At the end of the experiment, colonic Treg cells were analyzed by FACS.

### DSS-induced colitis model

Colitis was induced in mice by 2% dextran sulfate sodium (DSS) solution in drinking water. Mice were weighed daily and visually inspected for diarrhoea and rectal bleeding. The DAI was assessed blinded to the mouse groups (maximum total score 12). Histological activity score (maximum total score 40) was assessed as the sum of three parameters, extent, inflammation, and crypt damage ⁵⁵.

### TNBS-induced colitis model

2,4,6-trinitrobenzene sulfonic acid (TNBS) was obtained from Sigma-Aldrich. In order to presensitize mice, a 1.5 × 1.5 cm field of the abdominal skin was shaved, and 150 µl of a 1% (w/v) TNBS solution was applied. 7 days after presensitization mice were rechallenged intrarectally with 150 µl 2.5% TNBS in 50% ethanol under general anesthesia with isoflurane ⁵⁶. Sections of colon tissue were stained with H&E, and the histological score was determined as in the DSS model.

### Antibiotic treatment

To assess the possible contribution of gut microbiota in exacerbation of DSS-colitis by vagotomy, mice were treated with broad-spectrum antibiotics (6.7 g/L ampicillin, 6.7 g/L neomycin, 3.3 g/L vancomycin and 6.7 g/L metronidazole) via nasogastric tube (500 µL/ mouse) three times a week for 3 weeks. As a control, mice were treated with an identical dose of distilled water via nasogastric tube.

### In vivo administration of bethanechol, salbutamol, propranolol, methyllycaconitine, and GTS-21

Bethanechol (BETH) was dissolved in water. After surgery for 12 hours, mice were intraperitoneally injected daily, with water or BETH (300 µg per mouse) ⁵⁷. Salbutamol (β2-agonist) and propranolol (β-blocker) were used to assess the impact of adrenergic signaling on colonic Tregs homeostasis. Salbutamol and propranolol were dissolved in PBS. After surgery for 12 hours, mice were intraperitoneally injected daily, with PBS (200 µl per mouse), salbutamol (30 µg per mouse) or propranolol (300 µg per mouse) ⁵⁸. Methyllycaconitine (MLA, α7 nicotinic acetylcholine receptor antagonist) and GTS-21 (α7 nicotinic acetylcholine receptor agonist) were used to assess the role of the α7 nicotinic acetylcholine receptor on maintenance of colonic Treg. MLA and GTS-21 were dissolved in PBS. After surgery for 12 hours, mice were intraperitoneally injected daily, with PBS (200 µl per mouse), MLA (150 µg per mouse) or GTS-21 (300 µg per mouse) ⁵⁹.

### Retrograde tracing from liver

One microliters of Alexa Fluor 488 conjugated wheat germ agglutinin (WGA488) (5 mg/ml) were injected in liver using a 30-gauge needle connected to a Hamilton syringe at 40 spots. One week after WGA488 injection, mice were first perfused with PBS and then with 4% PFA in PBS. Isolated NG and DRG were post-fixed for 2 hours and cryoprotected by immersion with 30% sucrose in PBS for a further 24 hours. Fresh-frozen NG and DRG sections were cut 6-mm thick on a cryostat, collected on slides, and immediately dried. The slides were mounted with ProLong(TM) Diamond Antifade Mountant with DAPI. Electro-physiological recording of activity of sympathetic nerve

Sympathetic nerve activity measurements were performed as described previously ⁵⁷. In brief, the common hepatic branch of the vagus nerve or CG-SMG was identified and exposed to measure nerve activity. Electrical activity in each nerve was amplified 50,000 to 100,000 times with a band-pass filter of 100 to 1,000 kHz and monitored using an oscilloscope. The amplified and filtered nerve activity was converted to standard pulses by a window discriminator, which separated discharges from electrical background noise post-mortem. Both the discharge rates and the neurogram were sampled with a PowerLab analog-to-digital converter for recording and data analysis on a computer. Background noise, which was determined at 30 to 60 min after the animal was euthanized, was subtracted. Nerve activity was rectified and integrated with baseline nerve activity normalized to 100%.

### Isolation of colonic lamina propria mononuclear cells in mice

Lamina propria mononuclear cells (LPMC) isolation was performed as previously described ⁶. Dissected colon mucosa was cut into 5-mm pieces. Tissue was incubated with Ca²⁺, Mg²⁺-free HBSS containing 1 mM DTT and 5 µM EDTA at 37°C for 30 minutes, followed by further digestion with collagenase and DNase for 45 minutes. Cells were then separated with a Percoll density gradient. The numbers of live cells were determined by Countess II (Thermo Fisher Scientific).

### Isolation of splenocytes from mice

Spleens were smashed into 100-µm nylon and then erythrocytes lysed with 0.84% ammonium chloride solution.

### Enteric neurosphere-derived neurons

Total intestines from embryonic day 13.5 (E14.5) Wnt1-Cre/Floxed-EGFP double-transgenic were digested with 0.1% trypsin/EDTA trypsin for 30 minutes at 37°C. Cells were mechanically dissociated, wash and cultured in an ultra-low attachment T-25 cell culture flask (CORNING) for 7 days in a CO₂ incubator at 37°C in supplemented DMEM/F12 (25 µg/ml insulin, 100 µg/ml transferrin, 20 nM progesterone, 30 nM sodium selenate, 60 nM putrescine, 100 ng/ml recombinant human EGF, 100 ng/ml recombinant human FGF and 20 ng/ml B27 ⁵⁰). After neurosphere formation, enteric neurospheres were plated on non-coating cell culture plate and cultured for 7 days in the following differentiation medium (DMEM/F12 supplemented with 10% fetal bovine serum (FBS) and 1% penicillin-streptomycin (PS)). Cells differentiated from enteric neurosphere were dissociated using trypsin and stained with antibodies against PE-conjugated anti-mouse CD24 antibody (30F-1), APC-conjugated anti-mouse CD184 antibody (L276F12), PE/Cy7-conjugated anti-mouse/human CD44 antibody (IM7) and Brilliant Violet 510-conjugated anti-mouse CD45.2 antibody (104) for 30 minutes on ice. Cell sorting was performed using FACS aria II for collection of enteric neurosphere-derived neurons (GFP⁺CD45.2⁻CD184⁻CD44⁻CD24⁺ cells). For co-culture, sort-purified colonic APCs were added to the culture.

### Fluorescence-activated cell sorting (FACS) analysis

After blocking with anti-mouse CD16/CD32 antibody for 20 minutes, the cells were incubated with the specific fluorescence-labeled mAbs at 4°C for 30 minutes, followed by permeabilization with Permeabilization Buffer and intracellular staining with anti-Foxp3 mAb in case of Treg staining. The following mAbs were used for FACS analysis: anti-mouse CD45.2, CD3e, CD4, CD11b, CD11c, MHC-II, NK1.1, TCRβ, B220, NKp46, Gata3, IL-17A, IL-22, Foxp3, Helios and RORyt antibody. Dead Cells were excluded using 7-AAD stain or Fixable Viability Dye eFluor. Events were acquired with FACS Canto II (BD Biosciences) and analyzed with FlowJo software (BD Biosciences). Colonic APCs (CD45.2⁺CD3⁻NK1.1⁻B220⁻MHC-II⁺ cells), CD45⁺CD3⁻B220⁻NK1.1⁻CD11c⁺CD11b⁻, CD45⁺CD3⁻B220⁻NK1.1⁻CD11c⁺CD11b⁺ and CD45⁺CD3⁻B220⁻NK1.1⁻CD11c⁻CD11b⁺ cells) were sorted by BD FACS Aria-II (BD Bioscience). Colonic APCs were cultured in RPMI-1640 containing 10% fetal bovine serum and 1% penicillin-streptomycin for overnight, the cells were then stimulated with muscarine.

### Aldehyde dehydrogenase activity assay

Aldehyde dehydrogenase (ALDH) activity was determined using ALDEFLUOR staining kit according to the manufacturer's protocol. In brief, cells were suspended at a concentration of 10⁶ cells/ml in ALDEFLUOR assay buffer containing activated ALDEFLUOR substrate (final concentration of 1.5 µM) with or without the ALDH inhibitor diethylaminobenzaldehyde (DEAB) (final concentration of 15 µM) and incubated at 37°C for 30 minutes. FACS analysis was performed on a BD Biosciences FACS Canto II.

### In vitro Treg induction assay

Naive CD4⁺ cells were isolated from spleen in WT mice using naive CD4⁺ T cell isolation kit. Naive CD4⁺ cells (1 × 10⁵) were cultured in RPMI-1640 medium supplemented with 10% FBS, 2 mM glutamine, 100 U/ml penicillin, 100 lg/ml streptomycin, and 55 µM 2-mercaptoethanol in 96 well plate. For Treg induction, naive T cells were stimulated 2 µl/well anti-CD3/CD28 microbeads and 2 ng/ml TGF-β for 3 days with colonic APCs (2 × 10⁴) in the presence or absence of muscarine or neuro-spheroid derived neuron (1 × 10⁵) ⁶⁰.

### Tissue samples

Normal intestinal mucosa was obtained from unaffected areas of patients with colon cancer. All experiments were approved by the institutional review board of Keio University School of Medicine and written informed consent was obtained from all patients.

### Isolation of human colonic LP cells

Large intestines were dissected and cleaned *in situ* of mesenteric fat and connective tissue. The entire large intestine was cut into 0.5 cm pieces for digestion. These pieces were first washed in HBSS before incubation at 37°C for 20 minutes in PBS containing 1 mM DTT, and 5 mM EDTA. The supernatant, containing the IEL fraction, was discarded. The remaining LP fraction was then washed twice in PBS before digestion with 1.0 mg/ml collagenase and 0.05 mg/ml DNase for 60 minutes at 37°C. LP suspensions were passed through a 70-µm filter. Cells were then separated with a Percoll density gradient. The interface was collected and cells were washed before staining and cell sorting. For cell sorting, human colonic APC were gated on by selecting CD45⁺CD3⁻CD19⁻CD56⁻HLA-DR^{hi} cells were sorted using an BD FACS Aria-II. Human colonic APCs were cultured in RPMI-1640 containing 10% FSB and 1% penicillin-streptomycin for overnight, the cells were then stimulated with muscarine.

### RNA Sequencing analysis

RNA Sequencing (RNA-seq) was performed and analyzed as described previously ⁶¹. Total RNA was prepared from approximately 20,000 to 50,000 cells by using TRIzol. Total RNAs were subsequently processed to generate an mRNA-seq library using a NEBNext Poly(A) mRNA Magnetic Isolation Module (NEB, E7490S), NEBNext Ultra II Directional RNA Library Prep with Sample Purification Beads (NEB, E7765S) and NEBNext Multiplex Oligos for Illumina (Index Primers Set 1 and 2) (NEB, E7335S and E7550S) according to protocol. The libraries were sequenced for 150 bp (paired-end read) by Illumina. To quantify transcript abundance, the present inventor pseudo-aligned RNA-seq reads to ENSEMBL transcripts (release 95 GRCm38), using kallisto (v0.44.0, options: -b 100) ⁶². The present inventor visualized the expression levels of APC subset signature genes with neurotransmitter receptor genes (expression > 1 TPM in at least one sample) by creating heatmaps with hierarchically clustered rows and columns (MORPHEUS; https://software.broadinstitute.org/morpheus/) and the ternary plot (ggtern v3.1.0).

### Collection of fecal samples and bacterial DNA isolation

Fecal samples were collected sequentially on days 0 and 2 post surgery from the identical mice. In this particular experiment, each mouse was housed individually in a separate cage. Bacterial DNA was prepared as described previously ⁶³. In brief, bacterial DNA was isolated by the enzymatic lysis method using lysozyme and achromopeptidase. DNA samples were then purified by treating with ribonuclease A, followed by precipitation with 20% polyethylene glycol solution (PEG6000 in 2.5 M sodium chloride). DNA was then pelleted by centrifugation, rinsed with 75% ethanol, and dissolved in tris-ethylenediaminetetraacetic acid (trisEDTA) buffer. Sequencing and processing of bacterial 16S rRNA gene in fecal DNA

The hypervariable V3-V4 region of the 16S gene was amplified using Ex Taq Hot Start (Takara Bio) and subsequently purified using AMPure XP (Beckman Coulter). Mixed samples were prepared by pooling approximately equal amounts of each amplified DNA and sequenced using the Miseq Reagent Kit V3 (600 Cycle) and Miseq sequencer (Illumina), according to the manufacturer's instructions. Sequences were analyzed using the QIIME software package version 1.9.1 ^{64,65}. Paired-end sequences were joined using a fastq-join tool in the ea-utils software package (https://doi.org/10.2174/18750 36201307010001). High-quality sequences per sample (15,000) were randomly chosen from the quality filter-passed sequences. After trimming off both primer sequences using cutadapt (https://doi.org/10.14806/ej.17.1.200) followed by chimeras detection by the USEARCH ⁶⁶ de novo method, the sequences were assigned to operational taxonomic units using the UCLUST algorithm ⁶⁷ with a sequence identity threshold of 96%. Taxonomic assignments of each operational taxonomic unit were made by similarity searching against the publicly available 16S (RDP version 10.27 and CORE update 2 September 2012) and the NCBI genome database using the GLSEARCH program. The data were rarefied to 10,000 sequences per sample, as determined by the rarefaction curves. Relative abundances of the community members were determined using the rarefied data. UniFrac analysis was performed as described previously ⁶⁸.

### Gnotobiotic mice

Fecal samples from Sham and HVx mice were collected. Fecal samples were suspended in equal volumes (w/v) of PBS containing 40% glycerol, snap-frozen and stored at - 80°C until use. The frozen stocks were thawed, suspended in 5-fold volumes of PBS and passed through a 100-µm cell strainer. GF mice were orally inoculated with 200 µl of the suspensions using a sterile stainless-steel feeding needle. Phenotypes of colonic immune cells were analyzed after a colonization period of 3 weeks.

### Quantitative reverse-transcription (qRT-) PCR analysis

The present inventor isolated and purified RNA from colon tissues and cells using a RNeasy Mini Kit. Reverse transcription was carried out with an iScript cDNA Synthesis Kit. Real-time PCR amplification was performed using a Thermal Cycler Dice Real Time System (Takara Bio). Gene expression levels were normalized to 18S ribosomal mRNA.

### Histological and immunohistochemistry

Liver, colon, NG and DRG were fixed in 10% formalin and embedded in paraffin. Spinal cords (Th4-7 and 13) and colon were cryoprotected in a 30% sucrose solution for 24 hours and preserved in OCT compound. Paraffin-embedded colon sections were stained with H&E and then examined. For immunohistochemistry, antigens were activated by autoclaving and blocked using Block Ace. Primary antibody reaction was performed at room temperature for 4 hours (dilution ratio; PGP9.5 (1/1000), pERK1/2 (1/500), TUBB3 (1/200), TRPV1 (1/1000), TH (1/1000)) or overnight at 4°C (I-A/I-E (1/200), TUBB3 (1/200)). After washing with PBS, the sections were incubated with Alexa Fluor 488- or Alexa Fluor 647-labeled secondary antibodies (1/400) at room temperature for 2 hours. Tissue samples were observed under a BX53 microscope (Olympus) and an LSM 710 confocal laser scanning microscope (Carl Zeiss). The images were analyzed using Imaris (Oxford Instruments), ZEN (Carl Zeiss) and ImageJ (NIH).

### Immunohistochemistry for pERK1/2 and c-Fos

pEKR1/2 and c-Fos expression were analyzed immunohistochemically as reported ¹. DSS-treated mice were transcardially perfused with PBS including 4% paraformaldehyde and 0.2% picric acid under anesthesia. The nodose ganglions and brains were collected, post-fixed in the same fixative for 2 hours to overnight at 4°C and then incubated in phosphate buffer containing 30% sucrose for 48 hours. Longitudinal sections (8 µm) of NGs were cut with 48 µm intervals using a precision cryostat (Leica Microsystems, IL). Coronal sections (40 µm) of hindbrain were cut with 120 µm intervals using a freezing microtome. Rabbit polyclonal antibody to pERK1/2 (1/500) and Alexa 488-conjugated goat anti-rabbit IgG (1/500) were used. Fluorescence images were acquired with BX50 microscope and DP50 digital camera (Olympus). In c-Fos staining, anti-c-Fos antisera (1/10,000) were used as the primary antibody. Color was developed with nickel-diaminobenzidine (DAB). Neurons immunoreactive to pERK1/2 and c-Fos in medial NTS were counted.

### c-Fos immunostaining in the myenteric nerve plexus

To prepare myenteric nerve plexus, 3 cm of colon obtained from Sham, VGx or HVx mice in a fed state, were cut longitudinally and soaked in a plastic plate containing ice-cold PBS. The mucosal layer was removed and the myenteric nerve plexus was dropped into 4% PFA overnight, at room temperature washed with cold PBS. Samples were blocked for 1 hour at RT with blocking solution. Then samples were incubated overnight at RT with the primary antibodies (HuC/HuD, 1/500; c-Fos, 1/500) diluted in the antibody diluent solution, washed 3 times with PBS, incubated 90 minutes at RT with the secondary antibodies (1/400) and washed 3 times with PBS. Samples were mounted with fluorescent mounting medium. The fluorescence of different tissues was measured on confocal Zeiss Laser Scanning Microscope LSM-710. Measurement of CGRP, ACH and NE levels in colon

Neurotransmitter levels in colon were determined as previously described ⁶⁹⁻⁷¹. Colon tissues were wash with PBS and homogenized. The homogenates were centrifuged at 15,000 x g for 10 minutes at 4°C and supernatants were collected. Samples were kept at -80°C until use. Protein concentrations were determined by BCA assay (Thermo Fisher Scientific). CGRP (Phoenix Pharmaceuticals), acetylcholine (Abeam) and noradrenaline (LsBio) levels in homogenates were measured by ELISA.

### Western blot analysis.

Proteins were extracted from liver tissues using T-PER including protease inhibitor and PhosSTOP (Sigma). Western blotting was performed as previously described using Clarity Western ECL Substrate and the ChemiDoc Imaging System (Bio-Rad) ⁷².

### Knockdown of Raptor in the liver by RNA interference.

Si-negative control (Si-Cont) and Si-Raptor (In-VivoReady grade) were complexed with Invivofectamine 2.0 Reagent (Invitrogen) exactly according to the manufacturer's protocol. Subsequently, male WT mice (weighing 22-25 gram) were intravenously injected via the tail vein with 200 µl complexed siRNA at a dose of approximately 7 mg of siRNA per kg body weight.

### Statistics

All values are shown as mean ± s.e.m. Statistical analyses were performed using unpaired 2-tailed Student's t test or 1-way ANOVA and Tukey's post hoc test for multiple comparisons.

### Data availability

All raw and processed sequencing data herein is available via NCBI GEO under accession number GSE140952. All computer code to analyze RNA-seq is available at https://github.com/mikamiy/liver-brain-gut-neural-arc.

### [Description of Drawings]

Figure 1 Potential interaction between APCs and neurons in the gut.
a, Representative immunofluorescence staining images of CX3CR1-GFP (green) and β-tubulin III (red) in the murine colon. b, Representative CD11c and MHC-II staining of CD45.2⁺TCRβ⁻ CD3-B220-NK1.1 gated colonic lamina propria mononuclear cells from *Cx3cr1Gfp* mice. c-f, Eight-week-old male B6 mice (WT mice) were subjected to a sham-operation (Sham) or truncal vagotomy (VGx). Colonic T cell phenotypes and colonic gene expression were analyzed 2 days later (*n* = 12/group). c, Frequency of Foxp3⁺ cells (Treg) among CD4⁺ T cells in colonic lamina propria (LP). d, Expression of RORyt in colonic Foxp3⁺ Treg. e, Expression of *Aldh1a1* and *Aldhla2* mRNA in colonic APCs. f, Frequency of ALDH⁺ cells among MHC-II⁺ APCs (CD45⁺TCRβ⁻CD3⁻B220⁻NK1.1⁻MHC-II⁺) in the colon. Left panel, histograms of ALDH⁺ cells in APCs. Colonic mononuclear cells were incubated with ALDEFLUOR in the absence (filled) or presence (dotted line) of DEAB (ALDH inhibitor). The percent of Aldefluor⁺ cells is shown above the horizontal line indicating the positive gate. Right panel, quantification. g, Heat map of the expression of genes encoding neurotransmitter receptors, classified by sorted colonic and splenic APCs, as determined by RNA-seq analysis. h, Heat map of macrophage and dendritic cell marker genes for colonic CD11b⁺CD11c⁻ (CD11b SP), CD11b⁺CD11c⁺ (DP) and CD11b⁻CD11c⁺ (CD11c SP) cells. The sorting strategy for the experiment is shown in Figure 5. i, Ternary plot of gene expression in colonic CD11b SP, DP, and CD11c SP cells. The color scale indicates mRNA concentration. Neurotransmitter receptors and representative markers for macrophage and dendritic cell are shown. j, *Aldh1a1* and *Aldhla2* mRNA expression levels in colonic APCs treated with PBS (control), 10 µM acetylcholine (Ach), 10 µM muscarine (Mus), 100 nM adrenaline (Adre), 100 µM neuropeptide Y (NPY), 100 nM substance P (Sub-P), 10 µM serotonin (5-HT) or 100 ng/ml neuromedin U (NMU) for 12h (n = 5/group). k, *Aldh1a1* and *Aldhla2* expression in WT and mAChR TKO colonic APCs. Colonic APCs were isolated from WT or Chrm1,2,4-deficient (mAChR TKO) mice and treated with 10 µM Mus or left untreated for 12h (n = 6/group). l, *ALDH1A1* and *ALDH1A2* mRNA levels in human colonic APCs. Colonic APCs were treated with 10 µM Mus or left untreated for 12h (n = 7/group). Representative of three (a, b, j-l) independent experiments or pooled from three independent experiments (c-f). P values were obtained via unpaired two-tailed Student's t tests (c-f, k) or one-way ANOVA with Tukey's post hoc test (j-l). Error bars represent the mean ± s.e.m.

Figure 2 The hepatic vagal sensory afferent pathway is essential for NTS activation during colitis.
a, b, WT mice were subjected to a sham operation or HVx and then were given DSS for 7 days, starting at day 2 after surgery (n = 4/group). NTS, nucleus tractus solitarius; DMV, dorsal motor nucleus of the vagus; AP, area postrema; NG, nodose ganglion. a, Representative image of Immunostaining for c-Fos in medulla oblongata (upper panel, bar: 200 µm). Count for c-Fos expression in NTS and DMV per section (lower panel). b, Representative image of immunostaining for pERK in nodose ganglion (NG) (upper panel, bar: 100 µm). c, d, WGA retrograde tracing. Representative fluorescence images of Alexa Fluor 488⁺ neuron (green) and DAPI (blue) in NG at 1 week after injection of WGA in liver (c) and quantification (d). White arrows indicate Alexa Fluor 488⁺ neuron in NG. e, WT mice were subjected to Sham, VGx or hepatic vagotomy (HVx) (*n* = 9/group). f, WT mice were subjected to Sham, ventral subdiaphragmatic vagotomy (LVx) or dorsal subdiaphragmatic vagotomy (RVx) (*n* = 4/group). e, f, Frequency of Foxp3⁺ cells among CD4⁺ cells in colon at day 2 after surgery. Representative of two (a-d, f) independent experiments or pooled from three independent experiments (e). *P* values were obtained via unpaired two-tailed Student's *t* tests (a, b, d) or one-way ANOVA with Tukey's post hoc test (e, f). Error bars represent the mean ± s.e.m.

Figure 3 The liver-brain-gut axis regulates colonic Treg homeostasis through muscarinic signaling in APC.
a-d, WT and mAChR TKO mice were subjected to Sham or HVx. e, WT and mAchR TKO mice were subjected to Sham or HVx and injected additionally treated with bethanechol (BETH; i.p. 300 µg/day) every day. Phenotypes of colonic immune cell were analyzed at day 2 after surgery (*n* = 5/group). a, Frequency of ALDH⁺ cells among MHC-II⁺ colonic APCs. Histograms of ALDH⁺ cells with colonic APCs (left panel). Quantification (right panel). b, *Aldh1a1* and *Aldhla2* mRNA expression in colonic APC. c, e, Frequency of Foxp3⁺ cells among CD4⁺ cells in the colon. d, Frequency of RORγt⁺ cells among colonic Foxp3⁺ Tregs. *P* values were obtained via unpaired one-way ANOVA with Tukey's post hoc test. Error bars represent the mean ± s.e.m.

Figure 4 Perturbation of hepatic vagal afferents exacerbates murine colitis in a muscarinic signaling dependent manner.
a-c, WT mice were subjected to Sham or HVx and then were given DSS for 7 days, starting at day 2 after surgery. Graphs show pooled data from three independent experiments (*n* = 15/group). d-f, mAchR TKO mice were subjected to Sham or HVx and then were given DSS for 7 days, starting at day 2 after surgery. Graphs show pooled data from three independent experiments (*n* = 12/group). , d, Relative body weight change during acute colitis. b, e, DAI. c, f, Representative HE staining of colon sections (left panel, bar: 200 µm) and histological scores (right panel). *P* values were obtained via unpaired two-tailed Student's *t* tests. Error bars represent the mean ± s.e.m.

Figure 5 Muscarinic signaling in colonic APC activates induction of Treg.
a, Three-dimensional reconstruction of CX3CR1⁺ APC (green) and enteric neurons (purple) in colon in mice. b, Three-dimensional reconstruction of MHCII⁺ APC (green), enteric Tuj⁺ neurons (purple), and Foxp3⁺ Tregs (yellow). c, d, Anatomical diagram (c) and operative field (d) for subdiaphragmatic truncal vagotomy. e, f, Colonic T cell phenotypes in mice after VGx surgery. Representative contour plots with frequency of Foxp3⁺ cells (Treg) among CD4⁺ T cells in colonic LP (e) and RORγt⁺ pTregs in colonic Foxp3⁺ Tregs (f) . g, Expression of *Chrm1, Adrb2*, *Htr7* and *Chrna7* mRNA in colonic and splenic APCs. h, Strategy for sorting colonic CD11b⁻CD11c⁺ (CD11c SP), CD11b⁺CD11c⁺ (DP) and CD11b⁺CD11c⁻ (CD11b SP) subsets by FACS. i, j, Enteric neuron derived neuron-induced retinol metabolism-related gene expression in colonic APC was dependent on muscarinic signaling. i, Schema of experience. j, *Aldh1a1* and *Aldhla2* mRNA levels in colonic APC (*n* = 6/group). k, l, Muscarinic signaling in colonic APCs promoted induction of Treg. k, Schema of experience. l, Frequency of Foxp3⁺ Treg among CD4⁺ T cells (left panel). Representative contour plots (right panel). m, n, Enteric neuro-spheroid derived neuron enhanced Foxp3⁺ Treg induction via activation of muscarinic signaling in colonic APC. m, Schema of experience. n, Frequency of Foxp3⁺ Treg among CD4⁺ T cells (left panel). Representative contour plots (right panel). Quantification (*n* = 6/group). Representative of two (a, b, i-k) or three (e, f) independent experiments. *P* values were obtained via one-way ANOVA with Tukey's post hoc test. Data are shown as mean ± SEM (j, l, n).

Figure 6 Colitis activates liver-brain axis.
a-c, WT mice were subjected to Sham or VGx and then were given DSS for 7 days, starting at day 2 after surgery. Graphs show pooled data of three independent experiments (*n* = 15/group). a, Relative body weight change during colitis. ** indicates *p* < 0.01. b, DAI. c, Representative HE staining of colon sections (left panel, bar: 200 µm) and histological scores (right panel). d-f, WT mice were given DSS or water for 6 days (*n* = 6/group). d, Representative images of immunofluorescence staining for pERK1/2 (green) in NG (upper panel, bar: 100 µm). Quantification of pERK1/2-expressing neurons (lower panel). e, Representative images of c-Fos immunoreactivity in NTS (left panel, bar: 200 µm). Number of c-Fos immunoreactive neurons (right panel). f, Representative images of immunofluorescence double-staining for pERK1/2 (green) and PGP9.5 (red) in murine liver sections. Co-stained sites are shown in yellow (left panel). Scale bar indicates 10 µm. Quantification of pERK1/2-expressing area in PGP9.5 positive nerve fiber (right panel). g. Hepatic phosphor-mTOR and total mTOR protein levels. WT mice were treated with Abx-cocktail for 3 weeks and then were given DSS for 4 days. h, i, WT mice were intravenously injected with Si-negative control (Si-Contin) or Raptor (Si-Raptor) and at day 3 after were subjected to sham or HVx (*n* = 6/group). Phenotypes of colonic T cell were analyzed at day 2 after surgery. h, Frequency of Foxp3⁺ Treg among CD4⁺ T cells (left panel). Representative contour plots (right panel). i, Frequency of RORγt⁺ cells among colonic Foxp3⁺ Treg. Representative contour plots (left panel). Quantification (right panel). Representative of two independent experiments (d-i). *P* values were obtained via unpaired two-tailed Student's *t* tests (a-f) and one-way ANOVA with Tukey's post hoc test (h,i). Data are shown as mean ± SEM (b-f, h, i).

Figure 7 Anatomy of the hepatic vagus nerve in mice.
a, Anatomical diagram. b, Operative field for hepatic vagotomy. c, A schematic view showing the ignition of liver-brain-gut neural arc during colitis. d, The common hepatic branch of the vagus does not contain sympathetic nerve. Operative field for electrical recording of hepatic sympathetic nerve (left panel). Electrical activity in common hepatic branch of the vagus and hepatic sympathetic nerve, respectively (middle panel). Representative images of immunofluorescence staining for Tyrosine hydroxylase (TH) in hepatic branch and DRG (right panel, bar: 100 µm). e, Fluorescent immunohistochemistry of TRPV1⁺ neuron in hepatic vagal branch at day 2 after capsaicin application (bar: 200 µm). f, g, h, WGA retrograde tracing. f, g, WT mice were subjected to Sham or VGx and then were injected with Alexa Fluor 488 conjugated WGA at day 2 after surgery (n = 3/group). Fluorescence image of Alexa Fluor 488⁺ neuron (green) and DAPI (blue) in NG (f) and Th4 DRG (g) at 1 week after injection of WGA in liver.

Representative images (left panel, bar: 50 µm). h, Fluorescence images of Alexa Fluor 488⁺ neuron (green) and DAPI (blue) in DRG (Th4-7 and Th13) at 1 week after injection of WGA in liver (bar: 100 µm). Representative of two independent experiments (d-h). *P* values were obtained via unpaired two-tailed Student's *t* tests. Data are shown as mean ± SEM (f, g).

Figure 8 Effects of vagotomy on maintenance and stability of colonic pTreg.
WT mice were subjected to Sham, VGx or HVx (*n* = 9/group). Phenotypes and gene expression of colonic immune cell were analyzed at day 2 after surgery. a, Frequency of Foxp3⁺ cells among CD4⁺ cells in colon. Representative contour plots. b, Frequency of RORγt⁺ cells among Foxp3⁺ Treg in colonic LP. Representative contour plots (left panel). Quantification (right panel). c, Expression of *Aldh1a1* and *Aldhla2* mRNA in colonic APC. d, Frequency of ALDH⁺ cells among MHC-II⁺ colonic APCs. Histograms of ALDH⁺ cells with colonic APCs (left panel). Quantification (right panel). e, f, WT mice were subjected to Sham or HVx. e, f, WT mice were subjected to Sham or HVx, and colonic T cell phenotypes were analyzed at the indicated time point following HVx (*n* = 9/group). Frequency of Foxp3⁺ cells among CD4⁺ T cells in colonic LP (e) and RORγt⁺ cells among Foxp3⁺ Treg in colonic LP (f). Representative contour plots (left panel) and quantification (right panel) are shown (e, f). g, h, i, Frequency of Foxp3⁺ cells among CD4⁺ cells (g, h, i) and RORγt⁺ cells among colonic Foxp3⁺ Treg (g, h) in colon at day 2 after surgery. g, B6 mice were subjected to Sham or HVx (*n* = 10/group). h, BALB/c mice were subjected to Sham or HVx (*n* = 4/group). i, Six-week-old WT rats were subjected to Sham or HVx (*n* = 4/group). j, k, *Rag2*^{*-*/*-*} mice were subjected to Sham or HVx and then transferred with CD4⁺ CD45RB^{hi} T cells (*n* = 8/group). Four weeks after transfer, mice were sacrificed and colonic Treg cells were analyzed. j, Frequency of Foxp3⁺ cells among CD4⁺ cells in colon. Representative contour plots (left panel). Quantification (right panel). k, Frequency of RORγt⁺ cells among colonic Foxp3⁺ Treg. Representative contour plots (left panel).

Quantification (right panel). l, WT mice were subjected to Sham or HVx. Colonic immune cell phenotypes were analyzed 2 days later. Frequency of IFN-γ⁺, Gata3⁺ and IL-17A⁺ cells among CD4⁺ cells in colon (*n* = 9/group). Representative of two (e, f, h, i) independent experiments or pooled from three independent experiments (a-d, g, j-l). *P* values were obtained via one-way ANOVA with Tukey's post hoc test (b-f) and unpaired two-tailed Student's t tests (g-l). Data are shown as mean ± s.e.m.

Figure 9 Afferent vagal, but not spinal cord, activation from the liver is involved in colonic Treg homeostasis.
a-e, WT mice underwent either corn oil (Oil) or capsaicin (Cap) application to the hepatic vagal branch. Phenotypic analysis of T cell and APC in colon was performed at day 2 after application of capsaicin (c, d, *n* = 16/group; e, f, n = 8/group). a, b, Representative fluorescence images of TRPV1 (red) and DAPI (blue) in NG (a) and Th4-DRG (b). Scale bar indicates 100 µm. c, Frequency of Foxp3⁺ cells among CD4⁺ cells in colon. Representative contour plots (left panel). Quantification (right panel). d, Frequency of RORγt⁺ cells among colonic Foxp3⁺ Tregs. Representative contour plots (left panel). Quantification (right panel). e, Frequency of ALDH⁺ cells among MHC-II⁺ colonic APCs. Histograms of ALDH⁺ cells with colonic APCs (left panel). Quantification (right panel). f, Expression of *Aldh1a1* and *Aldhla2* mRNA in colonic APC. g-p, Eight-week-old WT type were intrathecally injected with capsaicin (g-j, *n* = 5/group) and resiniferatoxin (RTX) (k-p, *n* = 4/group). TRPV1⁺ nerves in spinal cord (Th4-7 and Th13) and colonic immune cells were analyzed at day 7 after administration. g, Fluorescent immunohistochemistry of TRPV1⁺ neuron in spinal cord (bar: 200 µm). Frequency of Foxp3⁺ cells among CD4⁺ cells (h, n) and RORγt⁺ cells among colonic Foxp3⁺ Tregs (i, o) in the colon. k, l, Effects of intrathecal injection of RTX in DRG (k) and NG (1). Scale bar indicates 100 µm. m, Colonic CGRP levels. Representative of two independent experiments (a-b, g-o) or pooled from two (e) or three (c, d, f) independent experiments. P values were obtained via unpaired two-tailed Student's t tests. Data are shown as mean ± s.e.m.

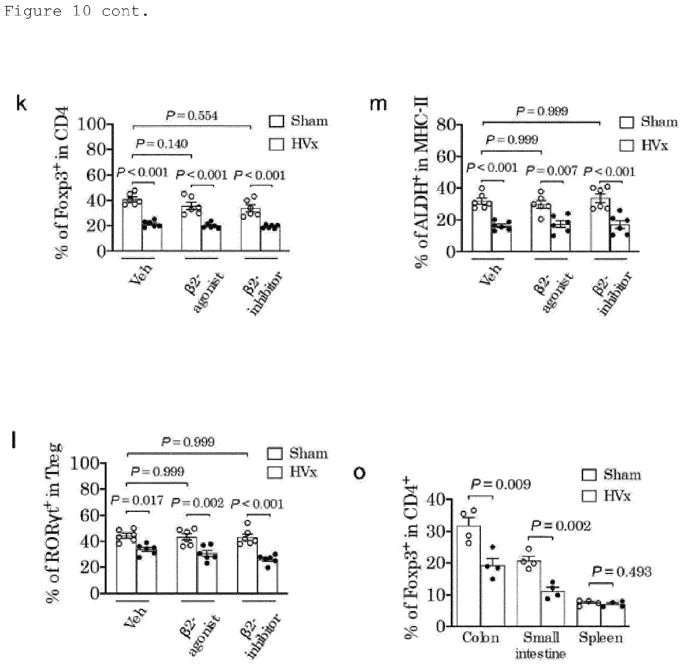

Figure 10 Hemi-subdiaphragmatic vagotomy revealed functional asymmetries of the vagus nerve.
a-c, WT mice were subjected to Sham, ventral (left) subdiaphragmatic vagotomy (LVx) or dorsal (right) subdiaphragmatic vagotomy (RVx) (*n* = 4/group). Phenotypic analysis of T cell and APC in colon was performed at day 2 after surgery. a, Frequency of Foxp3⁺ cells among CD4⁺ cells in colon. b, Frequency of RORγt⁺ cells among Foxp3⁺ Treg in colonic LP. Representative contour plots (left panel). Quantification (right panel). c, Frequency of ALDH⁺ cells among MHC-II⁺ colonic APCs. Histograms of ALDH⁺ cells with colonic APCs (left panel). Quantification (right panel). d-j, Ablation of sympathetic signaling via CG/SMG does not affect maintenance of Treg in colon. d, Operative field for CG/SMG ganglionectomy. e, Electrical activity in splanchnic nerve. The numbers in parentheses correspond to the nerves indicated in d. f-l, WT mice were subjected to Sham (n = 4) or ganglionectomy of CG/SMG (n = 5). j, WT mice were injected with MLA (α7-agonist, 150 µg/day, i.p.) for 2 days and then splenic T cells were analyzed at 12 hours after last injection (*n* = 5/group). f, i, j, Frequency of Foxp3⁺ cells among CD4⁺ cells in colon (f) and spleen (i, j). g, Frequency of RORγt⁺ cells among colonic Foxp3⁺ Tregs. h, Frequency of ALDH⁺ cells among MHC-II⁺ colonic APCs. k-m, WT mice were subjected to Sham or HVx and then injected daily with vehicle, salbutamol (30 µg/day, i.p.) or propanol (300 µg/day, i.p.) for 2 days (n = 6/group). Frequency of Foxp3⁺ cells among CD4⁺ cells (k), RORγt⁺ cells among colonic Foxp3⁺ Tregs (1), and ALDH⁺ cells among MHC-II⁺ colonic APCs (m) in the colon at 12 hours after the last injection. O, WT mice were subjected to Sham or HVx. T cell phenotypes in colon, small intestine and spleen were analyzed at day 2 after surgery. Frequency of Foxp3⁺ cells among CD4⁺ T cells in the colon, the small intestine, and the spleen (n = 4/group). Representative of two independent experiments (a-o). P values were obtained via one-way ANOVA with Tukey's post hoc test (b, c, k-o) and unpaired two-tailed Student's t tests (f-j). Data are shown as mean ± s.e.m.

Figure 11 Effects of VGx and HVx on intrinsic enteric neuron.
a-c, g, WT mice were subjected to Sham (n = 4) or VGx (n = 5). d-f, h, WT mice were subjected to Sham (n = 6) or HVx (n = 6). Activity of intrinsic enteric neuron were measured at day 2 after surgery. a, d, Representative images of immunofluorescence staining for HuC/D (white) and c-Fos (red) in colon. Scale bar indicates 100 µm. b, e, Quantification of c-Fos⁺ neurons. c, f, Expression of *Hand2* mRNA in colon. g, h, Colonic acetylcholine, norepinephrine and CGRP levels. * and ** indicate *p* < 0.05 and p < 0.01, respectively. P values were obtained via unpaired two-tailed Student's t tests. Data are shown as mean ± s.e.m.

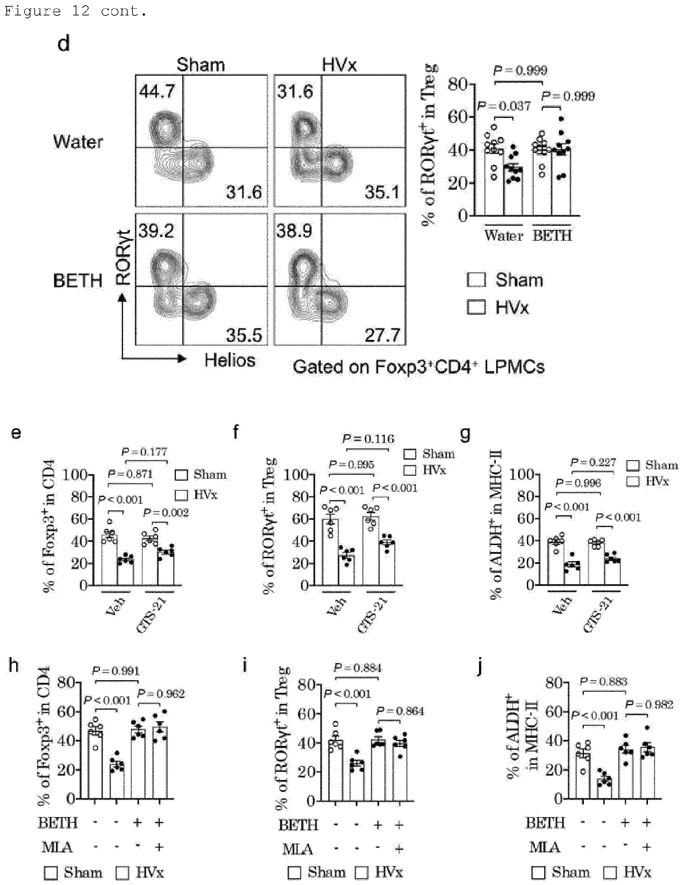

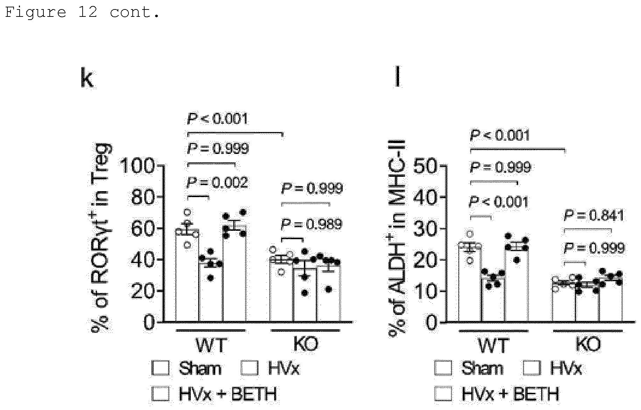

Figure 12 Effects of mAChR and α7nAChR on maintenance of colonic Treg.
a-g, WT mice were subjected to Sham or HVx and then injected daily with water or bethanechol (BETH; i.p. 300 µg/day) (a-d) or GST-21 (i.p. 300 µg/day) (e-g) for 2 days (a, b, n = 5/group; c, d, n = 10/group; e-g, n = 6/group). h-j, WT mice were subjected to Sham or HVx and then daily injected with water, BETH only or BETH⁺ MLA for 2 days (n = 6/group). k-l, WT and mAchR TKO mice were subjected to Sham or HVx and then daily injected with water or BETH for 2 days (n = 4/group). In relation to Figures 3c and 3d, phenotypes of colonic immune cell were analyzed at 12 hours after last injection. a, g, j, l, Frequency of ALDH⁺ cells among MHC-II⁺ colonic APCs. b, Expression of *Aldh1a1* and *Aldh1a2* mRNA in colonic APC. c, e, h, Frequency of Foxp3⁺ cells among CD4⁺ cells in colon. d, f, i, k, Frequency of RORγt⁺ cells among colonic Foxp3⁺ Tregs. P values were obtained via one-way ANOVA with Tukey's post hoc test. Data are shown as mean ± s.e.m.

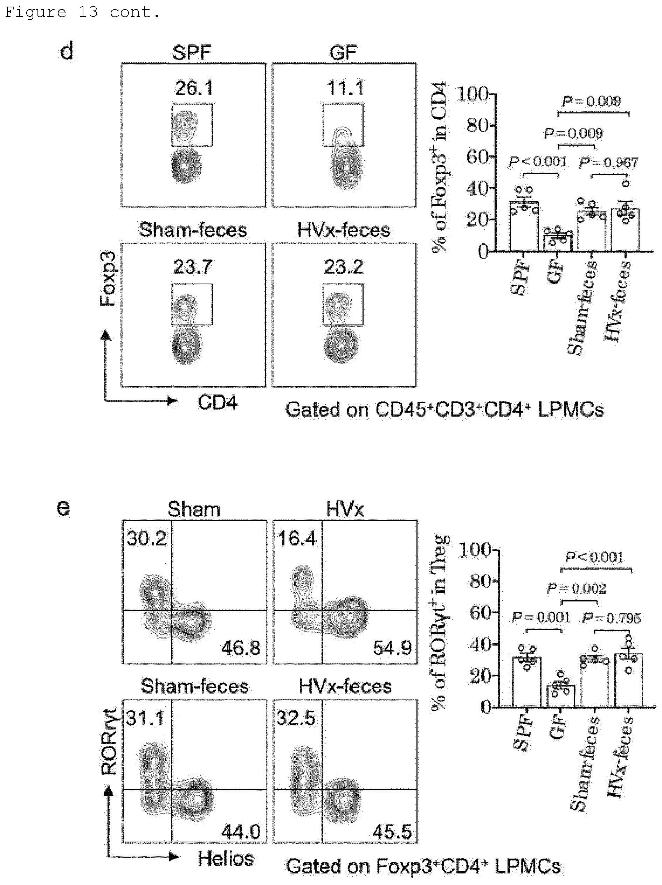

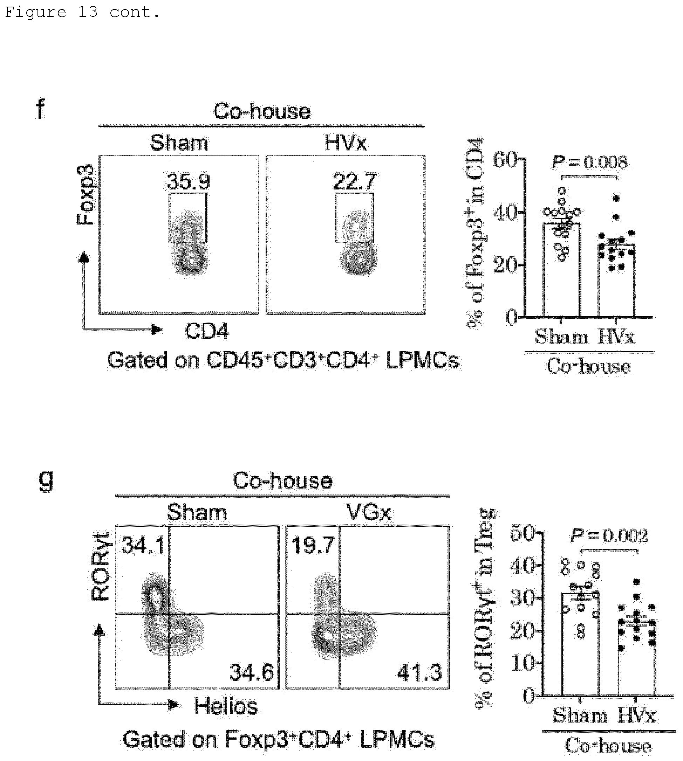

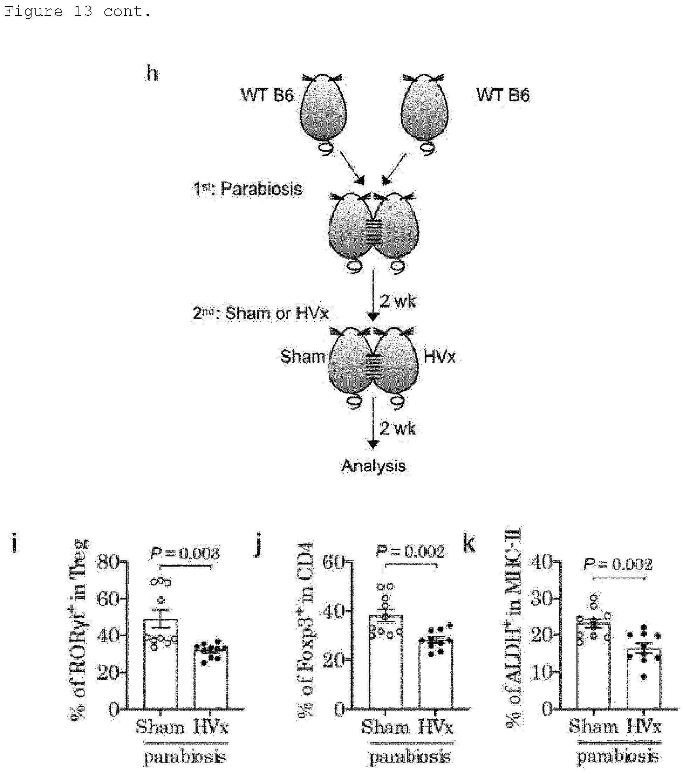

Figure 13 Effects of gut-microbiota on colonic Treg maintenance of the liver-brain-gut axis.
a-c, WT mice were subjected to Sham or HVx (n = 4/group). Fecal samples were collected on pre-treatment and days 2 post-surgery from the identical mice. a, α-diversity in the fecal microbiota. b, Principal coordinate analysis (PCoA) based on the weighted UniFrac analysis of bacterial community structures (black, pre-treatment; red, sham; blue, HVx). The two components of the weighted PCoA plot explained 45% and 22% of the variance. Dissimilarities between two groups were evaluated by permutational multivariate analysis of variance (PERMANOVA). c, Phylum-level taxonomic distribution. d, e, Fecal samples derived from sham-operated or HVx mice were inoculated into five-week-old male GF mice, followed by immunological phenotyping at day 21 post-inoculation (n = 5/group). f, g, WT mice were subjected to Sham or HVx and co-housed in the SPF facility of the inventor for 2 days. Graphs show pooled data of three independent experiments (n = 14/group). h-k, At day 14 post-parabiosis, mice were subjected to sham or HVx (n = 10/group). Phenotypes of colonic immune cell were analyzed 2 days later (h). l, m, WT mice were treated with Abx-cocktail (metronidazole, vancomycin, ampicillin and neomycin) for 3 weeks and subjected to Sham or HVx. Colonic T cell phenotypes were analyzed 2 days later. Graphs show pooled data of three independent experiments (n = 10/group). d, f, i, l, Frequency of Foxp3⁺ cells among CD4⁺ T cells in colon. e, g, j, m, Expression of RORγt⁺ in colonic Foxp3⁺ Treg. k, Frequency of ALDH⁺ cells among MHC-II⁺ colonic APCs. P values were obtained via one-way ANOVA with Tukey's post hoc test (a, d, e, l, m) or unpaired two-tailed Student's t tests (f, g, i-k). Data are shown as mean ± s.e.m.

Figure 14 Effects of HVx on colitis.
a-c, WT mice were sensitized with TNBS. After 7 days, mice were subjected to Sham or HVx, and treated with TNBS by intrarectal administration at the same time. Graphs show pooled data from three independent experiments (n = 20/group). d-f, Eight-week-old male *Rag2*^{*-*/*-*} mice were subjected to sham-operation or HVx and then were given DSS for 7 days, starting at day 2 after surgery (n = 12/group). g, h, Sham-operated and HVx mice were cohoused and orally challenged with 2.0% DSS (w/v) for 7 days. Graphs show pooled data of two independent experiments (n = 8/group). i, j, Abx treated mice were subjected to sham and HVx and after 2 days, orally challenged with 2.0% DSS (w/v) for 7 days. Graphs show pooled data of two independent experiments (n = 10/group). k, l, Sham operated and HVx *Myd88*-deficient mice were orally challenged with 2.0% DSS (w/v) for 7 days (n = 13/group). m-o, Sham-operated and hepatic vagotomized mice were orally challenged with 2.0% DSS (w/v) and daily treated with BETH for 7 days. Graphs show pooled data of two independent experiments (n = 10/group). a, d, g, I, k, m, Relative body weight change during acute colitis. b, e, h, j, l, n, DAI. c, f, o, Representative HE staining of colon sections (left panel, bar: 200 µm) and histological scores (right panel). Each experiment was repeated at least twice with similar results. P values were obtained via unpaired two-tailed Student's t tests. Error bars represent the mean ± s.e.m. p, A schematic view of the liver-brain-gut neural arc. A mouse in the supine position is illustrated. The liver senses the gut microenvironment and relays the sensory inputs to the left NTS of the brainstem, and ultimately to the left vagal parasympathetic nerves and enteric neurons. Gut APCs, activated by the liver-brain-gut neural arc, show enhanced ALDH expression and RA synthesis through mAChRs and maintain a reservoir of peripheral regulatory T cells.

### [References]

1. Pavlov, V. A. & Tracey, K. J. Neural regulation of immunity: molecular mechanisms and clinical translation. Nat. Neurosci. 20, 156-166 (2017).
2. Sharon, G., Sampson, T. R. & Geschwind, D. H. The Central Nervous System and the Gut Microbiome. Cell 167, 915-932 (2016).
3. Godinho-Silva, C., Cardoso, F. & Veiga-Fernandes, H. Neuro-Immune Cell Units: A New Paradigm in Physiology. Annu. Rev. Immunol. 37, 19-46 (2019).
4. Graham, D. B. Pathway paradigms revealed from the genetics of inflammatory bowel disease. Nature 578, 527-539 (2020).
5. Littman, D. R. & Rudensky, A. Y. Th17 and regulatory T cells in mediating and restraining inflammation. Cell 140, 845-858 (2010).
6. Honda, K. & Littman, D. R. The microbiota in adaptive immune homeostasis and disease. Nature 535, 75-84 (2016).
7. Jin, W. et al. Conversion of peripheral CD4+CD25-naive T cells to CD4+CD25+ regulatory T cells by TGF-beta induction of transcription factor Foxp3. J Exp Med 198, 1875-1886 (2003).
8. Mucida, D. et al. Reciprocal TH17 and regulatory T cell differentiation mediated by retinoic acid. Science 317, 256-260 (2007).
9. Smith, P. M. et al. The microbial metabolites, short-chain fatty acids, regulate colonic Treg cell homeostasis. Science 341, 569-573 (2013).
10. Mazmanian, S. K., Round, J. L. & Kasper, D. L. A microbial symbiosis factor prevents intestinal inflammatory disease. Nature 453, 620-625 (2008).
11. Belkaid, Y. & Hand, T. W. Role of the microbiota in immunity and inflammation. Cell 157, 121-141 (2014).
12. Coombes, J. L. & Powrie, F. Dendritic cells in intestinal immune regulation. Nat Rev Immunol 8, 435-446 (2008).
13. Kim, K. S. et al. Dietary antigens limit mucosal immunity by inducing regulatory T cells in the small intestine. Science 351, 858-863 (2016).
14. Chavan, S. S., Pavlov, V. A. & Tracey, K. J. Mechanisms and Therapeutic Relevance of Neuro-immune Communication. Immunity 46, 927-942 (2017).
15. Huh, J. R. & Veiga-Fernandes, H. Neuroimmune circuits in inter-organ communication. Nat Rev Immunol 20, 217-228 (2019).
16. Chu, C., Artis, D. & Chiu, I. M. Neuro-immune Interactions in the Tissues. Immunity 52, 464-474 (2020).
17. Veiga-Fernandes, H. & Mucida, D. Neuro-Immune Interactions at Barrier Surfaces. Cell 165, 801-811 (2016).
18. Coombes, J. L. et al. A functionally specialized population of mucosal CD103+ DCs induces Foxp3+ regulatory T cells via a TGF-beta and retinoic acid-dependent mechanism. J Exp Med 204, 1757-1764 (2007).
19. Sun, C.-M., Hall, J. A., Blank, R. B. & Belkaid, Y. Small intestine lamina propria dendritic cells promote de novo generation of Foxp3 T reg cells via retinoic acid. J Exp Med 204, 1775-1785 (2007).
20. Schulz, O. et al. Intestinal CD103 +, but not CX3CR1 +, antigen sampling cells migrate in lymph and serve classical dendritic cell functions. J Exp Med 206, 3101-3114 (2009).
21. Merad, M., Sathe, P., Helft, J. & Miller, J. The dendritic cell lineage: ontogeny and function of dendritic cells and their subsets in the steady state and the inflamed setting. Annu. Rev. Immunol. 31, 563-604 (2013).
22. Uematsu, S. et al. Regulation of humoral and cellular gut immunity by lamina propria dendritic cells expressing Toll-like receptor 5. Nat Immunol 9, 769-776 (2008).
23. Denning, T. L., Patel, S. R. & Williams, I. R. Lamina propria macrophages and dendritic cells differentially induce regulatory and interleukin 17-producing T cell responses. Nat Immunol 8, 1086-1094 (2007).
24. Berthoud, H.-R. Anatomy and function of sensory hepatic nerves. Anat Rec A Discov Mol Cell Evol Biol 280, 827-835 (2004).
25. Iwasaki, Y. et al. GLP-1 release and vagal afferent activation mediate the beneficial metabolic and chronotherapeutic effects of D-allulose. Nat Comms 9, 113 (2018).
26. Spadoni, I. et al. A gut-vascular barrier controls the systemic dissemination of bacteria. Science 350, 830-834 (2015).
27. Yoshimoto, S. et al. Obesity-induced gut microbial metabolite promotes liver cancer through senescence secretome. Nature 499, 97-101 (2013).
28. Uno, K. et al. A hepatic amino acid/mTOR/S6K-dependent signalling pathway modulates systemic lipid metabolism via neuronal signals. Nat Comms 6, 7940-15 (2015).
29. Polansky, J. K. et al. DNA methylation controls Foxp3 gene expression. Eur. J. Immunol. 38, 1654-1663 (2008).
30. Weiss, J. M. et al. Neuropilin 1 is expressed on thymus-derived natural regulatory T cells, but not mucosa-generated induced Foxp3+ T reg cells. J Exp Med 209, 1723-42- S1 (2012).
31. Josefowicz, S. Z., Lu, L.-F. & Rudensky, A. Y. Regulatory T cells: mechanisms of differentiation and function. Annu. Rev. Immunol. 30, 531-564 (2012).
32. Kanno, Y., Vahedi, G., Hirahara, K., Singleton, K. & O'Shea, J. J. Transcriptional and epigenetic control of T helper cell specification: molecular mechanisms underlying commitment and plasticity. Annu. Rev. Immunol. 30, 707-731 (2012).
33. Zhou, X. et al. Instability of the transcription factor Foxp3 leads to the generation of pathogenic memory T cells in vivo. Nat Immunol 10, 1000-1007 (2009).
34. Ohkura, N. Transcriptional and epigenetic basis of Treg cell development and function: its genetic anomalies or variations in autoimmune diseases. Cell Res. 133, 775-10 (2020).
35. Gabanyi, I. et al. Neuro-immune Interactions Drive Tissue Programming in Intestinal Macrophages. Cell 164, 378-391 (2016).
36. Moriyama, S. et al. β2-adrenergic receptor-mediated negative regulation of group 2 innate lymphoid cell responses. Science 359, 1056-1061 (2018).
37. Rosas-Ballina, M. et al. Acetylcholine-synthesizing T cells relay neural signals in a vagus nerve circuit. Science 334, 98-101 (2011).
38. Huston, J. M. et al. Splenectomy inactivates the cholinergic antiinflammatory pathway during lethal endotoxemia and polymicrobial sepsis. J Exp Med 203, 1623-1628 (2006).
39. Rosas-Ballina, M. et al. Splenic nerve is required for cholinergic antiinflammatory pathway control of TNF in endotoxemia. Proc. Natl. Acad. Sci. U.S.A. 105, 11008-11013 (2008).
40. Martelli, D., Farmer, D. G. S., McKinley, M. J., Yao, S. T. & McAllen, R. M. Anti-inflammatory reflex action of splanchnic sympathetic nerves is distributed across abdominal organs. Am. J. Physiol. Regul. Integr. Comp. Physiol. 316, R235-R242 (2019).
41. Karimi, K., Bienenstock, J., Wang, L. & Forsythe, P. The vagus nerve modulates CD4+ T cell activity. Brain Behav. Immun. 24, 316-323 (2010).
42. O'Mahony, C., van der Kleij, H., Bienenstock, J., Shanahan, F. & O'Mahony, L. Loss of vagal anti-inflammatory effect: in vivo visualization and adoptive transfer. Am. J. Physiol. Regul. Integr. Comp. Physiol. 297, R1118-26 (2009).
43. Di Giovangiulio, M. et al. Vagotomy affects the development of oral tolerance and increases susceptibility to develop colitis independently of the alpha-7 nicotinic receptor. Mol. Med. 22, 464-476 (2016).
44. Frolkis, A. D. et al. Depression increases the risk of inflammatory bowel disease, which may be mitigated by the use of antidepressants in the treatment of depression. Gut 68, 1606-1612 (2019).
45. Zhao, C.-M. et al. Denervation suppresses gastric tumorigenesis. Sci Transl Med 6, 250ra115-250ra115 (2014).
46. Han, W. et al. A Neural Circuit for Gut-Induced Reward. Cell 175, 665-678.e23 (2018).
47. Glass, C. K., Saijo, K., Winner, B. & Marchetto, M. C. Mechanisms underlying inflammation in neurodegeneration. Cell 140, 918-934 (2010).
48. Schroeder, B. O. & Backhed, F. Signals from the gut microbiota to distant organs in physiology and disease. Nat Med 22, 1079-1089 (2016).
49. Bonaz, B. et al. Chronic vagus nerve stimulation in Crohn's disease: a 6-month follow-up pilot study. Neurogastroenterol. Motil. 28, 948-953 (2016).
50. Nagoshi, N. et al. Ontogeny and multipotency of neural crest-derived stem cells in mouse bone marrow, dorsal root ganglia, and whisker pad. Cell Stem Cell 2, 392-403 (2008).
51. Madisen, L. et al. A robust and high-throughput Cre reporting and characterization system for the whole mouse brain. Nat. Neurosci. 13, 133-140 (2010).
52. Dezfuli, G. et al. Subdiaphragmatic vagotomy with Pyloroplasty Ameliorates the obesity caused by genetic deletion of the Melanocortin 4 receptor in the mouse. Front Neurosci 12, 104 (2018).
53. Yamada, M., Terayama, R., Bando, Y., Kasai, S. & Yoshida, S. Regeneration of the abdominal postganglionic sympathetic system. Neurosci. Res. 54, 261-268 (2006).
54. Mikami, Y. et al. Competition between colitogenic Th1 and Th17 cells contributes to the amelioration of colitis. Eur. J. Immunol. 40, 2409-2422 (2010).
55. Hayashi, A. et al. A single strain of Clostridium butyricum induces intestinal IL-10-producing macrophages to suppress acute experimental colitis in mice. Cell Host Microbe 13, 711-722 (2013).
56. Wirtz, S. et al. Chemically induced mouse models of acute and chronic intestinal inflammation. 12, 1295-1309 (2017).
57. Kimura, K. et al. Central Insulin Action Activates Kupffer Cells by Suppressing Hepatic Vagal Activation via the Nicotinic Alpha 7 Acetylcholine Receptor. Cell Rep 14, 2362-2374 (2016).
58. Mohammadpour, H. et al. β2 adrenergic receptor-mediated signaling regulates the immunosuppressive potential of myeloid-derived suppressor cells. J. Clin. Invest. 129, 5537-5552 (2019).
59. Yamamoto, T. et al. Anti-allergic role of cholinergic neuronal pathway via α7 nicotinic ACh receptors on mucosal mast cells in a murine food allergy model. PLoS ONE 9, e85888 (2014).
60. Fantini, M. C., Dominitzki, S., Rizzo, A., Neurath, M. F. & Becker, C. In vitro generation of CD4+ CD25+ regulatory cells from murine naive T cells. 2, 1789-1794 (2007).
61. Iwata, S. et al. The Transcription Factor T-bet Limits Amplification of Type I IFN Transcriptome and Circuitry in T Helper 1 Cells. Immunity 46, 983-991.e4 (2017).
62. Bray, N. L., Pimentel, H., Melsted, P. & Pachter, L. Near-optimal probabilistic RNA-seq quantification. Nat Biotechnol 34, 525-527 (2016).
63. Nakamoto, N. et al. Commensal Lactobacillus Controls Immune Tolerance during Acute Liver Injury in Mice. Cell Rep 21, 1215-1226 (2017).
64. Caporaso, J. G. et al. QIIME allows analysis of high-throughput community sequencing data. Nat. Methods 7, 335-336 (2010).
65. Kuczynski, J. et al. Using QIIME to analyze 16S rRNA gene sequences from microbial communities. Curr Protoc Bioinformatics Chapter 10, Unit 10.7.-10.7.20 (2011).
66. Edgar, R. C., Haas, B. J., Clemente, J. C., Quince, C. & Knight, R. UCHIME improves sensitivity and speed of chimera detection. Bioinformatics 27, 2194-2200 (2011).
67. Edgar, R. C. Search and clustering orders of magnitude faster than BLAST. Bioinformatics 26, 2460-2461 (2010).
68. Tsuda, A. et al. Influence of Proton-Pump Inhibitors on the Luminal Microbiota in the Gastrointestinal Tract. Clin Transl Gastroenterol 6, e89 (2015).
69. de Jong, P. R. et al. TRPM8 on mucosal sensory nerves regulates colitogenic responses by innate immune cells via CGRP. Mucosal Immunol 8, 491-504 (2015).
70. Ghia, J.-E., Blennerhassett, P. & Collins, S. M. Impaired parasympathetic function increases susceptibility to inflammatory bowel disease in a mouse model of depression. J. Clin. Invest. 118, 2209-2218 (2008).
71. Houlden, A. et al. Brain injury induces specific changes in the caecal microbiota of mice via altered autonomic activity and mucoprotein production. Brain Behav. Immun. 57, 10-20 (2016).
72. Teratani, T. et al. Aortic carboxypeptidase-like protein, a WNT ligand, exacerbates nonalcoholic steatohepatitis. J. Clin. Invest. 128, 1581-1596 (2018).

### [Example 2]

### [Description of Drawings]

Figure 15 Schema of vagal hepatic nerve stimulation (VHNS).

C57BL6/J mice (male, 10 weeks old) were purchased. The mice were acclimatized for 1 week and then laparotomized under inhalation anesthesia, and a cuff electrode (Figure 15a) was placed in the hepatic branch of the vagus nerve in the mice (Figure 15b). Minus and plus poles were set on the liver and brain sides, respectively, such that current would flow from the liver to brain sides. After electrode placement, about 1-week recovery period was established. The electrode was then connected to a modular stimulator, and freely moving mice were given electrical stimulation under conditions of 10 Hz, pulse width 500 µs, ON 10s/OFF 90s, and 3h/day. A control group was set to mice in which the electrode was placed in the hepatic branch and connected to a modular stimulator, albeit without stimulation. After given electrical stimulation for 3 consecutive days, the mice were slaughtered, and the large intestine and the brain were collected. The constituent ratio of regulatory T cells (Tregs) in the colon was analyzed by FACS (Figure 15c). VHNS increased colonic Tregs. An activated region in the brain by electrical stimulation was evaluated with the neural activity marker cFos as an index. As a result of confirming a *cFos* mRNA expression region by *in situ* hybridization, activated left nucleus tractus solitarius (NTS), to which the hepatic branch of the vagus nerve is projected, left dorsal nucleus of vagus nerve (DMV), to which cells of efferent vagus nerve belong, and area postrema (AP) were observed (Figure 15d).

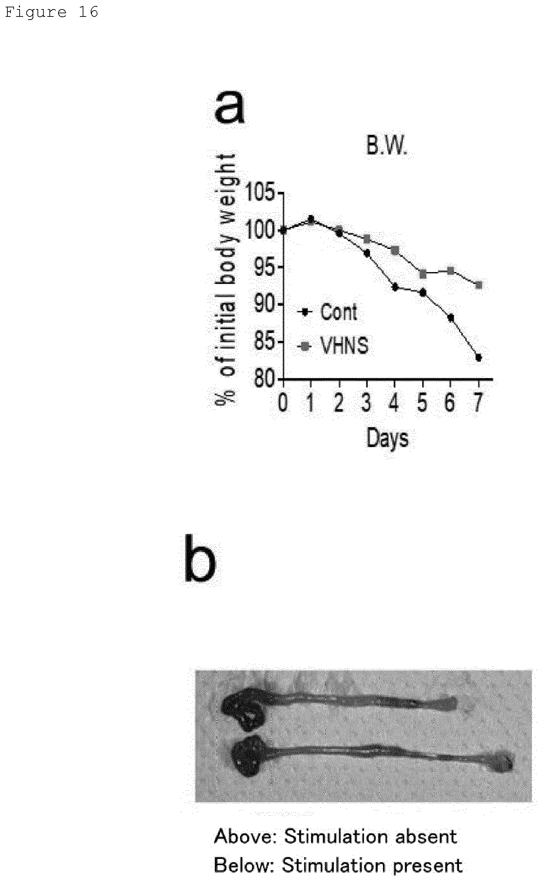

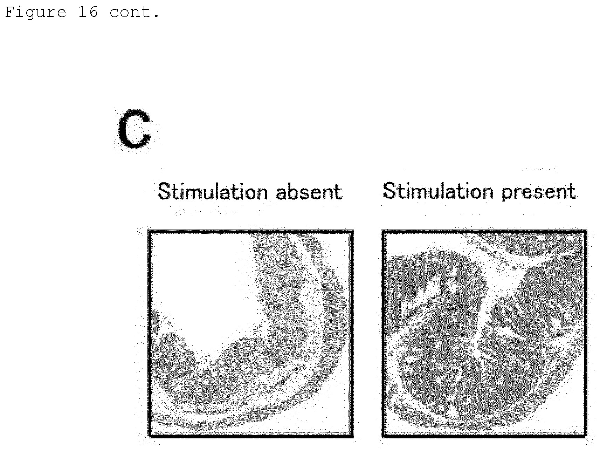

Figure 16 Vagal hepatic nerve stimulation (VHNS) inhibits pathological condition of colitis in mice.

C57BL6/J mice (male, 10 weeks old) were purchased. The mice were acclimatized for 1 week and then laparotomized under inhalation anesthesia, and a cuff electrode was placed in the hepatic branch of the vagus nerve in the mice. Minus and plus poles were set on the liver and brain sides, respectively, such that current would flow from the liver to brain sides. After electrode placement, about 1-week recovery period was established. For colitis development, the mice were then given 2% dextran sulfate sodium (DSS) solution in drinking water. At the same time with the start of DSS administration, the electrode was connected to a modular stimulator, and freely moving mice were given electrical stimulation under conditions of 10 Hz, pulse width 500 µs, ON 10s/OFF 90s, and 3h/day. A control group was set to mice in which the electrode was placed in the hepatic branch and connected to a modular stimulator, albeit without stimulation. The large intestine was collected from the mice 7 days after DSS administration, and the pathological condition of colitis was evaluated with pathological preparations and intestinal lengths as indexes. As a result of measuring body weight over time for 7 days following colitis development, VHNS inhibited weight loss ascribable to DSS colitis (Figure 16a). VHNS also inhibited intestinal shortening ascribable to DSS colitis (Figure 16b). VHNS that inhibited the colitis-induced dropout of large intestine epithelial cells was observed in histopathological preparations (Figure 16c).

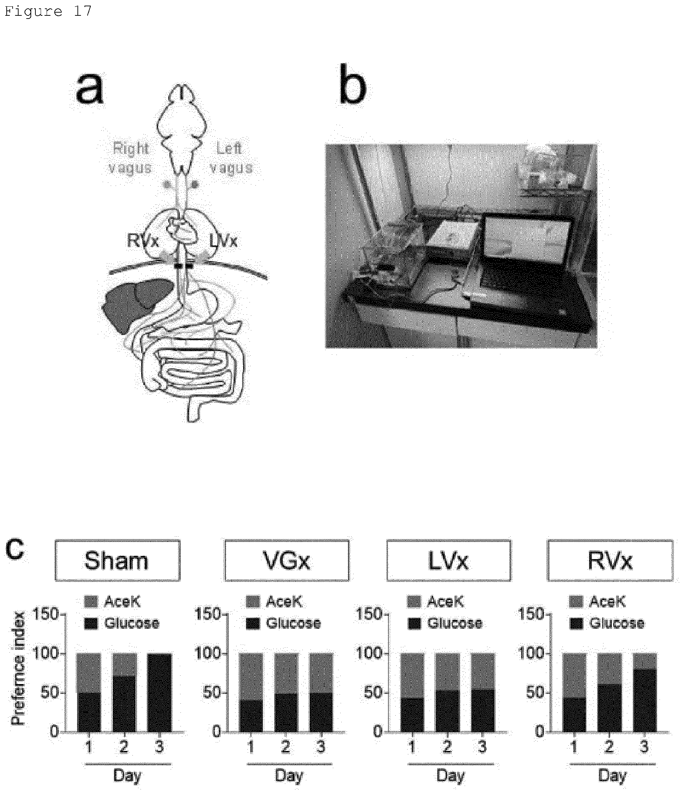

Figure 17 Two-bottle preference assays using vagotomized mice.

C57BL6/J mice (male, 10 weeks old) were purchased. The mice were acclimatized for 1 week, then laparotomized under inhalation anesthesia, and vagotomized bilaterally (VGx), at the left vagus (LVx) or at the right vagus (RVx) (Figure 17a). Laparotomized mice (Sham) were used as a control. About 1-week recovery period was established, and two-bottle preference assays were then carried out using these mice. Two drinking bottles were prepared, one of which was filled with a 600 mM aqueous glucose solution (Glu) and the other of which was filled with a 30 mM aqueous solution of the artificial sweetener acesulfame potassium (Ace K). These bottles were placed in a lick testing apparatus for preference analysis (Figure 17b) to perform the experiment. The mice mentioned above were raised from PM8 to AM10 in a cage in the apparatus, and the number of touches of the mouth to the drinking bottle was counted. This test was repeated in the identical individuals for 3 days, and the ratio between sucrose and Ace K in the total number of touches was calculated every day. This ratio was evaluated as preference. At the day of initiation of the test (Day 1), preference was almost the same between sucrose and Ace K in all mice. However, Sham and RVx mice selectively ingested sucrose with each passing day. On the other hand, VGx and LVx mice chose to equally ingest sucrose and Ace K even at day 3 of the test (Figure 17c).

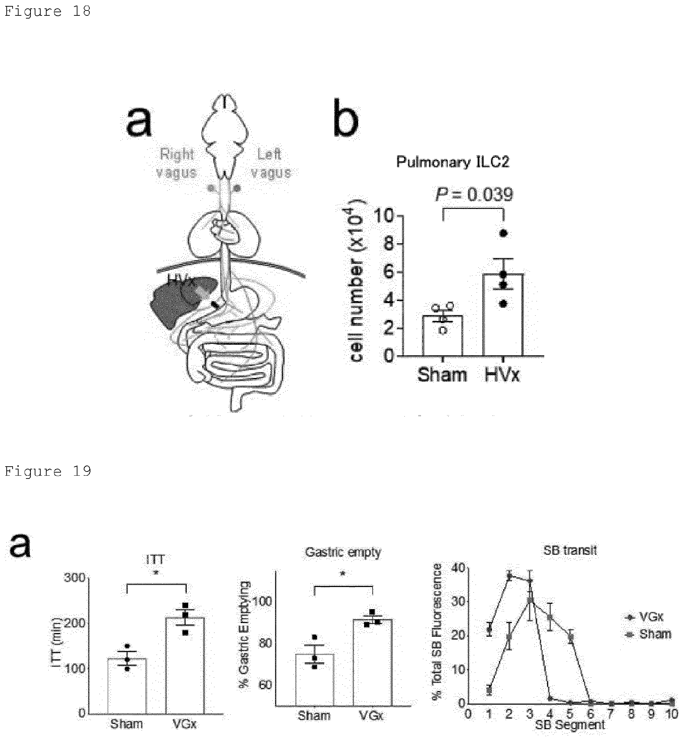

Figure 18 Regulation of pulmonary immune cells by hepatic branch of vagus nerve.

C57BL6/J mice (male, 10 weeks old) were purchased. The mice were acclimatized for 1 week and then laparotomized under inhalation anesthesia, and hepatic vagotomy (HVx) was performed in the mice (Figure 18a). Laparotomized mice (Sham) were used as a control. About 1-week recovery period was established, and immune cells were then collected from the lung in these mice. The number of innate lymphoid cells 2 (ILC2) in the lung was analyzed by FACS. The number of pulmonary ILC2 cells was increased by HVx (Figure 18b).

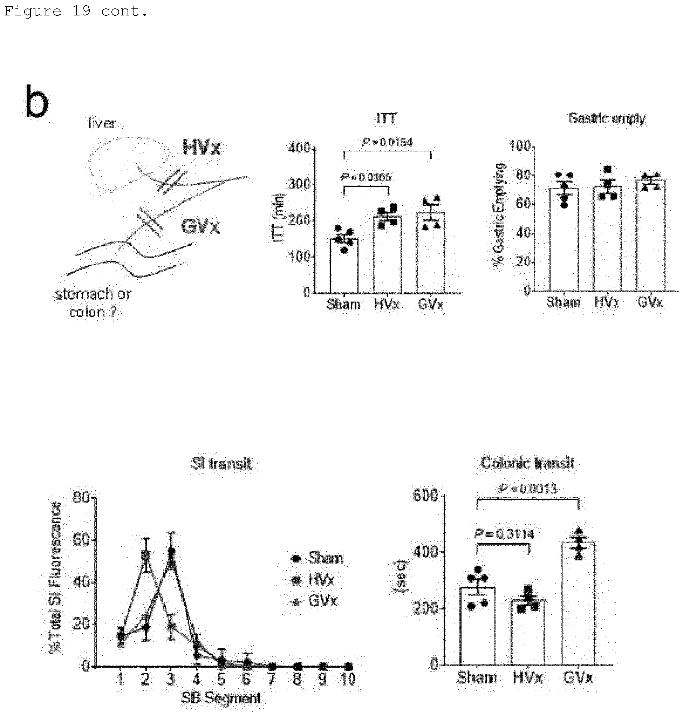

Figure 19 Regulation of intestinal peristalsis by vagus nerve.

(Figure 19a) C57BL6/J mice (male, 10 weeks old) were purchased. The mice were acclimatized for 1 week and then laparotomized under inhalation anesthesia, and bilateral vagotomy (HVx) was performed in the mice. Laparotomized mice (Sham) were used as a control. About 1-week recovery period was established, and these mice were then evaluated for intestinal peristaltic activity. The motility of the whole gastrointestinal tract was evaluated by the intestinal transit time (ITT) test. The motility of the stomach was evaluated by the gastric empty test. The motility of the small intestine was evaluated by the small-bowel (SB) transit test. Vagotomy reduced gastric and small intestinal motility and suppressed gastrointestinal peristalsis.

(Figure 19b) C57BL6/J mice (male, 10 weeks old) were purchased. The mice were acclimatized for 1 week and then laparotomized under inhalation anesthesia, and vagotomy of the proper hepatic branch (HVx) or the gastroduodenal branch (GVx) was performed in the mice (Figure 19b). Laparotomized mice (Sham) were used as a control. About 1-week recovery period was established, and these mice were then evaluated for intestinal peristaltic activity. The motility of the whole gastrointestinal tract was evaluated by the intestinal transit time (ITT) test. The motility of the stomach was evaluated by the gastric empty test. The motility of the small intestine was evaluated by the small-bowel (SB) transit test. The motility of the large intestine was evaluated by the colonic transit test. Neither HVx nor GVx affects gastric motility. HVx reduced small intestinal motility, whereas GVx reduced large intestinal motility. These results suggest that the vagus nerve regulates the peristaltic activity of a gastrointestinal region differing among branches.

All publications, patents and patent applications cited herein are incorporated herein by reference in their entirety.

### [Industrial Applicability]

The present invention is applicable in industry related to medicine.

## Claims

1. A therapeutic agent for a disease, comprising a substance having an effect of regulating an amount of peripheral regulatory T cells in the gut, wherein the substance is a substance activating or inhibiting afferent pathway of the hepatic branch of the vagus nerve, a substance activating or inhibiting efferent pathway of the left vagus nerve, or an agonist or an antagonist of a muscarinic acetylcholine receptor.

2. The therapeutic agent for a disease according to claim 1, wherein the disease is an inflammatory bowel disease, an autoimmune disease, an allergy, a cancer, depression, or a gastrointestinal infection.

3. The therapeutic agent for a disease according to claim 1, wherein the disease is an inflammatory bowel disease.

4. The therapeutic agent for a disease according to any one of claims 1 to 3, wherein the substance having an effect of regulating an amount of peripheral regulatory T cells in the gut is an agonist of a muscarinic acetylcholine receptor.

5. The therapeutic agent for a disease according to claim 4, wherein the agonist of a muscarinic acetylcholine receptor is bethanechol, muscarine, pilocarpine, or cevimeline.

6. The therapeutic agent for a disease according to any one of claims 1 to 3, wherein the substance having an effect of regulating an amount of peripheral regulatory T cells in the gut is an antagonist of a muscarinic acetylcholine receptor.

7. The therapeutic agent for a disease according to claim 6, wherein the antagonist of a muscarinic acetylcholine receptor is atropine, tropicamide, oxybutynin, propiverine, tolterodine, solifenacin, or imidafenacin.

8. A method for screening for a therapeutic agent for a disease, comprising the steps of: co-culturing intestinal antigen-presenting cells and CD4 positive T cells in the presence of a test substance; and detecting induction of regulatory T cells.

9. The screening method according to claim 8, wherein the induction of regulatory T cells is detected by detection of expression of FoxP3.

10. The screening method according to claim 8 or 9, wherein the disease is an inflammatory bowel disease, an autoimmune disease, an allergy, a cancer, depression, or a gastrointestinal infection.

11. The screening method according to claim 8 or 9, wherein the disease is an inflammatory bowel disease.

12. A method for treating a disease by regulating an amount of peripheral regulatory T cells in the gut, comprising activating or inhibiting afferent pathway of the hepatic branch of the vagus nerve in a subject to be treated, activating or inhibiting efferent pathway of the left vagus nerve in a subject to be treated, or administering an agonist or an antagonist of a muscarinic acetylcholine receptor to a subject to be treated.

13. The method for treating a disease according to claim 12, wherein the disease is an inflammatory bowel disease, an autoimmune disease, an allergy, a cancer, depression, or a gastrointestinal infection.

14. The method for treating a disease according to claim 12, wherein the disease is an inflammatory bowel disease.

15. The method for treating a disease according to any one of claims 12 to 14, comprising administering an agonist of a muscarinic acetylcholine receptor to a subject to be treated.

16. The method for treating a disease according to claim 15, wherein the agonist of a muscarinic acetylcholine receptor is bethanechol, muscarine, pilocarpine, or cevimeline.

17. The method for treating a disease according to any one of claims 12 to 14, comprising administering an antagonist of a muscarinic acetylcholine receptor to a subject to be treated.

18. The method for treating a disease according to claim 17, wherein the antagonist of a muscarinic acetylcholine receptor is atropine, tropicamide, oxybutynin, propiverine, tolterodine, solifenacin, or imidafenacin.

19. The method for treating a disease according to any one of claims 12 to 18, wherein the subject to be treated is a non-human animal.

20. A method for operating a cuff electrode, comprising stimulating the hepatic branch of the vagus nerve to regulate an amount of peripheral regulatory T cells in the gut.
